(19) Europäisches Patentamt
European Patent Office
Office européen des brevets

(11) Veröffentlichungsnummer: **0 343 487 B1**

(12) **EUROPÄISCHE PATENTSCHRIFT**

(45) Veröffentlichungstag der Patentschrift: **20.01.93**

(21) Anmeldenummer: **89108806.4**

(22) Anmeldetag: **17.05.89**

Die Akte enthält technische Angaben, die nach dem Eingang der Anmeldung eingereicht wurden und die nicht in dieser Patentschrift enthalten sind.

(51) Int. Cl.5: **C07C 69/63**, C09K 19/30, C09K 19/34, C07C 69/65, G02F 1/13, C07D 239/26, C07D 213/24

(54) **Alpha-Fluorcarbonsäurecyclohexylester.**

(30) Priorität: **26.05.88 CH 1989/88**
**05.04.89 CH 1248/89**

(43) Veröffentlichungstag der Anmeldung:
**29.11.89 Patentblatt 89/48**

(45) Bekanntmachung des Hinweises auf die Patenterteilung:
**20.01.93 Patentblatt 93/03**

(84) Benannte Vertragsstaaten:
**CH DE GB IT LI**

(56) Entgegenhaltungen:
**WO-A-87/05017**
**GB-A- 2 182 037**

(73) Patentinhaber: **F. HOFFMANN-LA ROCHE AG**
**Postfach 3255**
**CH-4002 Basel(CH)**

(72) Erfinder: **Buchecker, Richard, Dr.**
**Felsenstrasse 10**
**CH-8008 Zürich(CH)**
Erfinder: **Kelly, Stephen, Dr.**
**Salinenstrasse 3A**
**CH-4313 Möhlin(CH)**
Erfinder: **Leenhouts, Frans, Dr.**
**Violaweg 73**
**CH-4303 Kaiseraugst(CH)**

(74) Vertreter: **Cottong, Norbert A. et al**
**Grenzacherstrasse 124 Postfach 3255**
**CH-4002 Basel(CH)**

EP 0 343 487 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Verbindungen, deren Herstellung, flüssigkristalline Gemische, insbesondere Gemische mit chiral getilteter smektischer Phase, die solche Verbindungen enthalten, sowie die Verwendung für elektro-optische Zwecke.

Flüssige Kristalle eignen sich als Dielektrika in Anzeigevorrichtungen, da ihre optischen Eigenschaften durch eine elektrische Spannung beeinflusst werden können. Geeignete elektro-optische Vorrichtungen sind dem Fachmann gut bekannt. Beispiele solcher Vorrichtungen sind Zellen mit dynamischer Streuung, DAP-Zellen (Deformation aufgerichteter Phasen), TN-Zellen (twisted-nematic) und STN-Zellen (super twisted-nematic) mit verdrillt nematischer Struktur, Gast/Wirt-Zellen, Phase-Change-Zellen mit einem cholesterisch-nematischen Phasenübergang und SBE-Zellen (super birefringence effect).

In Appl. Phys. Lett. 36, 899 (1980) und in Recent Developments in Condensed Matter Physics 4, 309 (1981) werden ferner elektro-optische Vorrichtungen auf der Basis von chiral getilteten smektischen Flüssigkristallen vorgeschlagen. Hierbei werden die ferroelektrischen Eigenschaften dieser Materialien ausgenutzt. Als getiltete smektische Phasen eignen sich beispielsweise smektisch C, F, G, H, I und K Phasen. Bevorzugt sind im allgemeinen smektisch C Phasen, welche besonders grosse Ansprechgeschwindigkeiten ermöglichen. Die chiral getilteten Phasen werden üblicherweise mit $S_C^*$, $S_F^*$ etc. bezeichnet, wobei der Stern die Chiralität angibt.

Ferroelektrische Flüssigkristalle sollten eine gute Stabilität gegenüber chemischen und thermischen Einflüssen und gegenüber elektrischen Feldern aufweisen. Ferner sollten sie eine geeignete Mesophase über einen breiten Temperaturbereich, niedere Viskosität und insbesondere eine ausreichend hohe spontane Polarisation besitzen.

Verbindungen, welche für obengenannte Zwecke in Frage kommen könnten, sind beispielsweise die in GB-A-2 182 037 oder in WO-A-8 705 017 spezifisch offenbarten Phenylester von verzweigten α-chlorierten aliphatischen Carbonsäuren.

Als ferroelektrische Flüssigkristalle eignen sich vorzugsweise Gemische bestehend aus einem oder mehreren optisch aktiven Dotierstoffen und einem Flüssigkristallmaterial, welches aus einer oder mehreren Komponenten bestehen kann und in der Regel eine getiltete smektische Phase aufweisen sollte. Die optisch aktiven Dotierstoffe müssen nicht selbst smektisch sein, sollten aber im Flüssigkristallmaterial eine chiral getiltete smektische Phase erzeugen und eine hohe spontane Polarisation induzieren. Um eine hohe spontane Polarisation im Gemisch zu erzielen und/oder die Menge an optisch aktivem Dotierstoff relativ niedrig halten zu können, sind Dotierstoffe erwünscht, die bereits in geringen Mengen eine hohe spontane Polarisation zu induzieren vermögen.

Anderseits sollte die Ganghöhe (Pitch) des Flüssigkristalls deutlich grösser sein als der Plattenabstand der verwendeten Zelle und typischerweise mindestens etwa 10 µm betragen, um gut schaltende, bistabile Displays zu erhalten. Optisch aktive Dotierstoffe für ferroelektrische Flüssigkristalle sollten daher vorzugsweise im Gemisch eine chiral getiltete smektische Phase mit hoher spontaner Polarisation aber vergleichsweise kleiner Verdrillung (d.h. mit grosser Ganghöhe) induzieren.

Die Erfindung betrifft optisch aktive Verbindungen der allgemeinen Formel

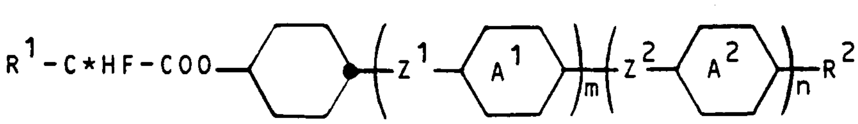

I

worin m für die Zahl 1 steht und $R^2$ eine unsubstituierte oder mit Halogen substituierte Alkyl- oder Alkenylgruppe bezeichnet, in welcher gegebenenfalls eine Methylengruppe durch Sauerstoff ersetzt ist und/oder gegebenenfalls eine Methylengruppe durch eine Estergruppe -COO- oder -OOC- ersetzt ist; oder m für die Zahl 0 oder 1 steht und $R^2$ eine Gruppe der allgemeinen Formel

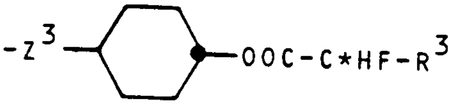

II

bezeichnet; $R^1$ und $R^3$ unabhängig voneinander eine Alkyl- oder Alkenylgruppe darstellen, in welcher gegebenenfalls eine Methylengruppe durch Sauerstoff ersetzt ist; $Z^1$, $Z^2$ und $Z^3$ unabhängig voneinander eine einfache Kovalenzbindung, $-CH_2O-$, $-OCH_2-$, $-CH_2CH_2-$, -COO- oder -OOC- bedeuten; n für die Zahl 0 oder 1 steht; die Ringe $A^1$ und $A^2$ unabhängig voneinander unsubstituiertes oder mit Halogen, Cyano und/oder Methyl substituiertes 1,4-Phenylen bedeuten, in welchem gegebenenfalls 1 oder 2 CH-Gruppen durch Stickstoff ersetzt sind; und $C^*$ ein chirales Kohlenstoffatom bezeichnet; mit der Massgabe, dass nicht zugleich $R^1$ eine Alkylgruppe, m und n die Zahl 1, $Z^1$ die Gruppe $-CH_2CH_2-$ oder $-CH_2O-$ und Ring $A^1$, $Z^2$, Ring $A^2$ und $R^2$zusammen 4-(5-Alkyl-2-pyrimidinyl)phenyl bedeuten.

Die erfindungsgemässen Verbindungen sind farblos, in üblichen Flüssigkristallmaterialien gut löslich und besitzen in der Regel selbst flüssigkristalline Eigenschaften. Obwohl der chirale $\alpha$-Fluorcarbonsäurerest an eine gesättigte Gruppe gebunden ist, induzieren die Verbindungen der Formel I in getilteten smektischen Phasen eine erstaunlich hohe spontane Polarisation. Anderseits wird aber im Gemisch überraschenderweise nur eine vergleichsweise geringe Verdrillung induziert. Im Gegensatz zu $\alpha$-Fluorcarbonsäurephenylestern besitzen die vorliegenden $\alpha$-Fluorcarbonsäurecyclohexylester zudem eine sehr gute Stabilität.

Der Ausdruck "unsubstituiertes oder mit Halogen, Cyano und/oder Methyl substituiertes 1,4-Phenylen, in welchem gegebenenfalls 1 oder 2 CH-Gruppen durch Stickstoff ersetzt sind" umfasst in Rahmen der vorliegenden Erfindung Gruppen wie 1,4-Phenylen, Fluor-1,4-phenylen, Chlor-1,4-phenylen, Brom-1,4-phenylen, Cyano-1,4-phenylen, 2,3-Dicyano-1,4-phenylen, 2,3-Difluor-1,4-phenylen, Methyl-1,4-phenylen, Pyridin-2,5-diyl, Pyrimidin-2,5-diyl, Pyrazin-2,5-diyl, Pyridazin-3,6-diyl und dergleichen.

Der Ausdruck "Halogen" umfasst Fluor, Chlor, Brom und Jod, insbesondere Fluor, Chlor und Brom.

Der Ausdruck "unsubstituierte oder mit Halogen substituierte Alkyl- oder Alkenylgruppe, in welcher gegebenenfalls eine Methylengruppe durch Sauerstoff ersetzt ist und/oder gegebenenfalls eine Methylengruppe durch eine Estergruppe -COO- oder -OOC- ersetzt ist" umfasst unsubstituierte oder mit Halogen (insbesondere Fluor oder Chlor) substituierte Alkyl-, Alkenyl-, Alkoxy-, Alkenyloxy-, Alkoxyalkyl-, Alkoxycarbonyl-, Alkenyloxycarbonyl-, Alkanoyloxy-, Alkenoyloxy-, Alkooxyalkoxycarbonyl-, Alkoxyalkanoyloxygruppen und dergleichen, insbesondere Alkyl, Alkenyl, Alkoxy, Alkenyloxy, Alkoxycarbonyl, Alkenyloxycarbonyl, Alkanoyloxy, Alkenoyloxy, 2-Fluoralkyl, 2-Chloralkyl, 2-Fluoalkoxy, 2-Chloralkoxy, 2-Fluoralkoxycarbonyl, 2-Chloalkoxycarbonyl, 2-Fluoralkanoyloxy und 2-Chloralkanoyloxy. Ein gegebenenfalls vorhandenes Sauerstoffatom und eine gegebenenfalls vorhandene Estergruppe können zusammen eine Carbonatgruppe bilden; vorzugsweise sind sie jedoch nicht direkt benachbart. Die Gruppen können geradkettig oder verzweigt sein (z.B. n-Alkyl, n-Alkoxy, Isoalkyl, Isoalkoxy) und die Radikalstelle in 1-Stellung (sofern nicht ausdrücklich angegeben, ist im folgenden die Radikalstelle jeweils in 1-Stellung) oder in der Kette, z.B. in 2-Stellung (wie 2-Alkyl, 2-Alkoxy, 2-Alkoxycarbonyl), aufweisen. Die Gruppen besitzen vorzugsweise höchstens 15 Kohlenstoffatome, beispielsweise 2-15 Kohlenstoffatome. Beispiele bevorzugter Gruppen sind Methyl, Aethyl, Propyl, Butyl, Pentyl, Hexyl, Heptyl, Octyl, Nonyl, Decyl, Isopropyl, Isobutyl, 2-Butyl, 2-Octyl, 2-Methylbutyl, 3-Methylpentyl, Vinyl, 1-Propenyl, 1-Butenyl, 1-Pentenyl, Allyl, 2-Butenyl, 2-Pentenyl, 3-Butenyl, 3-Pentenyl, 4-Pentenyl, 5-Hexenyl, 6-Heptenyl, Methoxy, Aethoxy, Propyloxy, Butyloxy, Pentyloxy, Hexyloxy, Heptyloxy, Octyloxy, Nonyloxy, Decyloxy, Isopropyloxy, Isobutyloxy, 3-Butyloxy, 2-Octyloxy, 2-Methylbutyloxy, 3-Methylpentyloxy, Allyloxy, 2E-Butenyloxy, 3-Butenyloxy, 4-Pentenyloxy, 5-Hexenyloxy, 6-Heptenyloxy, 2-Fluor-1-propyloxy, 2-Chlor-1-propyloxy, 2-Fluor-1-butyloxy, 2-Chlor-1-butyloxy, 2-Fluorpropanoyloxy, 2-Chlorpropanoyloxy, 2-Fluorbutanoyloxy, 2-Chlorbutanoyloxy, 2-Fluorpentanoyloxy, 2-Chlorpentanoyloxy, 2-Fluorhexanoyloxy, 2-Chlorhexanoyloxy, 2-Fluorheptanoyloxy, 2-Chlorheptanoyloxy, 2-Fluoroctanoyloxy, 2-Chloroctanoyloxy, 2-Octyloxycarbonyl und dergleichen.

Der Ausdruck "Allyl- oder Alkenylgruppe, in welcher gegebenenfalls eine Methylengruppe durch Sauerstoff ersetzt ist" umfasst Alkyl, Alkenyl, Alkoxy, Alkenyloxy, Alkoxyalkyl, Alkenyloxyalkyl und Alkoxyalkenyl, insbesondere Alkyl. Alkenyl, Alkoxyalkyl und Alkenyloxyalkyl. Die Gruppen können geradkettig oder verzweigt sein und die Radikalstelle in 1-Stellung oder in der Kette aufweisen. Die Gruppen besitzen vorzugsweise etwa 1-15 Kohlenstoffatome, insbesondere etwa 3-15 Kohlenstoffatome. Beispiele bevorzugt Gruppen sind die oben für Alkyl, Alkenyl etc. angegebenen Beispiele. Im allgemeinen sind geradkettige Reste bevorzugt, insbesondere die geradkettigen Reste, welche die Radikalstelle in 1-Stellung oder 2-Stellung aufweisen.

Wenn n in obiger Formel I die Zahl 1 bedeutet, steht zweckmässigerweise m für die Zahl 1 ($m = 0$, $n = 1$ umfasst die gleichen Verbindungen wie $m = 1$, $n = 0$).

Bedeutet $R^2$ eine Gruppe der Formel II, so besitzen die beiden $\alpha$-Fluorcarbonsäurereste vorzugsweise die gleiche Konfiguration am $\alpha$-C-Atom.

Die obige Formel I umfasst somit optisch aktive Verbindungen der allgemeinen Formeln

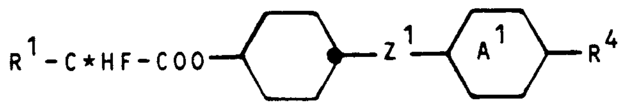

Ia

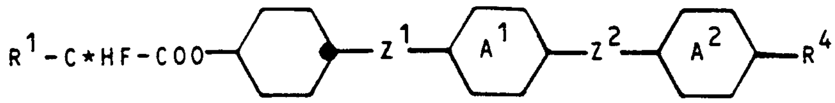

Ib

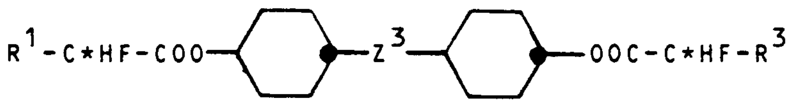

Ic

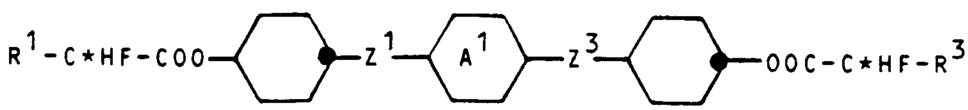

Id

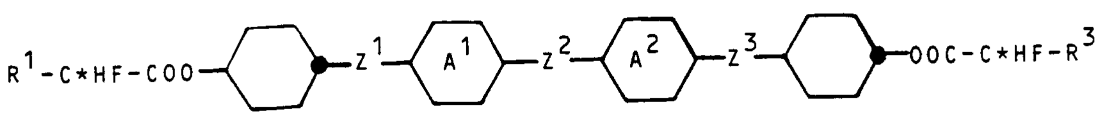

Ie

worin $R^1$, $R^3$, $Z^1$, $Z^2$, $Z^3$, $C^*$ und die Ringe $A^1$ und $A^2$ die obigen Bedeutungen haben; $R^4$ eine unsubstituierte oder mit Halogen substituierte Alkyl- oder Alkenylgruppe bezeichnet, in welcher gegebenenfalls eine Methylengruppe durch Sauerstoff ersetzt ist und/oder gegebenenfalls eine Methylengruppe durch eine Estergruppe -COO- oder -OOC- ersetzt ist; mit der Massgabe, dass in Formel Ib nicht zugleich $R^1$ eine Alkylgruppe, $Z^1$ die Gruppe $-CH_2CH_2-$ oder $-CH_2O-$ und Ring $A^1$, $Z^2$, Ring $A^2$ und $R^4$ zusammen 4-(5-Alkyl-2-pyrimidinyl)-phenyl bedeuten.

Die Ringe $A^1$ und $A^2$ in obigen Formeln I, Ia, Ib, Id und Ie bedeuten unabhängig voneinander vorzugsweise 1,4-Phenylen, Fluor-1,4-phenylen, Chlor-1,4-phenylen, Brom-1,4-phenylen, Cyano-1,4-phenylen, 2,3-Difluor-1,4-phenylen, Pyridin-2,5-diyl oder Pyrimidin-2,5-diyl. Von den Verbindungen der Formeln I, Ib und Ie sind im allgemeinen diejenigen besonders bevorzugt, worin einer oder beide der Ringe $A^1$ und $A^2$ 1,4-Phenylen, Pyrimidin-2,5-diyl, Fluor-1,4-phenylen, Chlor-1,4-phenylen, 2,3-Difluor-1,4-phenylen, und/oder Cyano-1,4-phenylen, insbesondere 1,4-Phenylen bedeuten. Besonders bevorzugt sind in der Regel diejenigen Verbindungen der Formeln I und Ia-Ie, worin einer der Ringe $A^1$ und $A^2$ 1,4-Phenylen, Fluor-1,4-phenylen oder 2,3-Difluor]-1,4-phenylen bedeutet und der andere der Ringe $A^1$ und $A^2$ 1,4-Phenylen oder Pyrimidin-2,5-diyl bedeutet.

Beispiele bevorzugter Gruppen erfindungsgemässer Verbindungen sind die optisch aktiven Verbindungen der allgemeinen Formeln

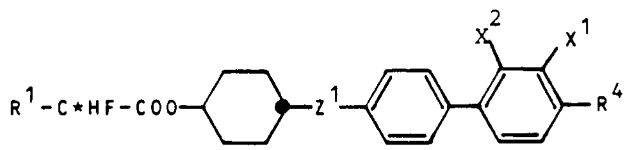

I-1

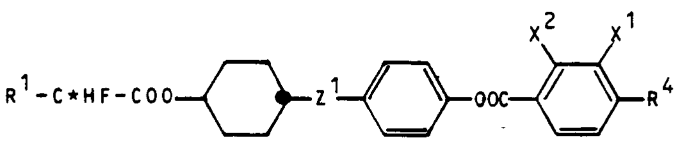

I-2

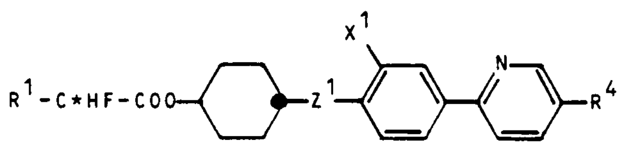

I-3

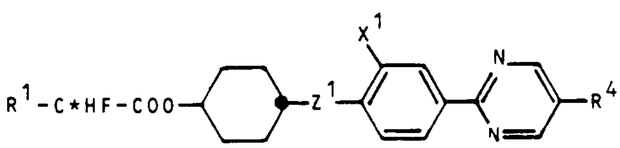

I-4

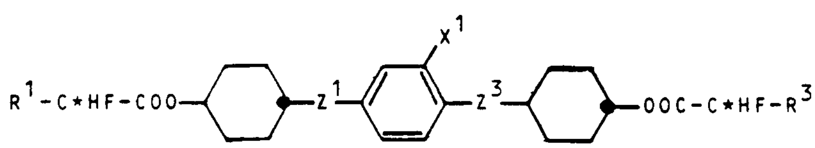

I-5

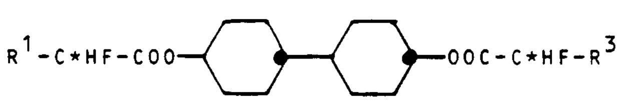

I-6

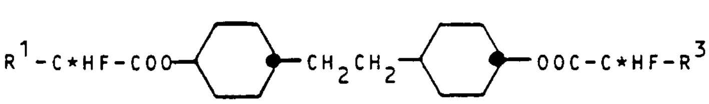

I-7

worin $R^1$, $R^3$, $R^4$, $Z^1$, $Z^3$ und $C^*$ die obigen Bedeutungen haben; $X^1$ und $X^2$ Wasserstoff, Fluor, Chlor, Brom, Cyano oder Methyl bedeuten; mit der Massgabe, dass in Formel I-4 $X^1$ Fluor, Chlor, Brom, Cyano oder Methyl bezeichnet, wenn $Z^1$ $-CH_2CH_2-$ oder $-CH_2O-$ darstellt.

$Z^1$ in den obigen Formeln I, Ia, Ib, Id, Ie und I-1 bis I-5 bedeutet jeweils vorzugsweise eine einfache Kovalenzbindung, $-CH_2O-$, $-CH_2CH_2-$ oder -COO-. $Z^3$ in den obigen Formeln II, Id, Ie und I-5 bedeutet jeweils vorzugsweise eine einfache Kovalenzbindung, $-OCH_2-$, $-CH_2CH_2-$ oder -OOC-. $Z^2$ in den obigen Formeln I, Ib und Ie bedeutet vorzugsweise eine einfache Kovalenzbindung, -COO- oder -OOC-. Ferner steht in den Formeln I-1 und I-2 vorzugsweise mindestens einer der Substituenten $X^1$ und $X^2$ für Wasserstoff oder Fluor; besonders bevorzugt sind in der Regel diejenigen Verbindungen, worin $X^1$ Wasserstoff, Fluor, Chlor, Brom oder Cyano und $X^2$ Wasserstoff bedeuten, sowie diejenigen Verbindungen, worin $X^1$ Wasserstoff oder Fluor und $X^2$ Fluor bedeuten.

Bevorzugte Reste $R^1$ in den obigen Formeln I, Ia bis Ie und I-1 bis I-7 sind Alkyl, Alkenyl, Alkoxyalkyl und Alkenyloxyalkyl, insbesondere Alkyl. Die Reste $R^1$ können geradkettig oder verzweigt sein und besitzen im allgemeinen höchstens 15 Kohlenstoffatome, vorzugsweise 3-15 Kohlenstoffatome und besonders bevorzugt 3-8 Kohlenstoffatome. Beispiele bevorzugter Reste $R^1$ sind Propyl, Isopropyl, Butyl, 2-Butyl, Pentyl, 2-Pentyl, Allyl, 3-Butenyl, 4-Pentenyl, Alkoxymethyl wie Methoxymethyl oder Aethoxymethyl, Alkenyloxymethyl wie Allyloxymethyl oder (3-Butenyl)oxymethyl, 1-Alkoxyäthyl wie 1-Methoxyäthyl oder 1-Aethoxyäthyl, 1-Alkenyloxyäthyl wie 1-Allyloxyäthyl oder 1-(3-Butenyl)oxyäthyl, 2-Alkoxyäthyl wie 2-methoxyäthyl oder 2-Aethoxyäthyl, 2-Alkenyloxyäthyl wie 2-Allyloxyäthyl oder 2-(3-Butenyl) oxyäthyl und dergleichen.

Bevorzugte Reste $R^3$ in den obigen Formeln II, Ic, Id, Ie und I-4 bis I-7 sind Alkyl, Alkenyl, Alkoxyalkyl und Alkenyloxyalkyl, insbesondere Alkyl. Die Reste $R^3$ können geradkettig oder verzweigt sein und besitzen im allgemeinen höchstens 15 Kohlenstoffatome, vorzugsweise 3-15 Kohlenstoffatome und besonders bevorzugt 3-8 Kohlenstoffatome. Beispiele bevorzugter Reste für $R^3$ sind die oben zu $R^1$ angegebenen Reste. $R^1$ und $R^3$ können verschiedene oder vorzugsweise auch gleiche Bedeutung haben.

Gegebenenfalls vorhandene chirale Reste $R^1$ bzw. $R^3$ können vorzugsweise ebenfalls in optisch aktiver Form vorliegen. Vorzugsweise wird jene Konfiguration gewählt, die die spontane Polarisation der Verbindung verstärkt.

Besonders bevorzugte Reste $R^1$ und $R^3$ sind 1-Alkyl und 2-Alkyl (= 1-Methylalkyl). 2-Alkylreste besitzen vorzugsweise S-Konfiguration, wenn die benachbarte Gruppe -C*HF- die S-Form aufweist, und R-Konfiguration, wenn die benachbarte Gruppe -C*HF- die R-Form aufweist.

$R^2$ in obiger Formel I bedeutet einen Rest $R^4$ oder eine Gruppe der Formel II. In Formel II besitzen $R^3$ und $Z^3$ vorzugsweise eine der oben angegebenen bevorzugten Bedeutungen.

Bevorzugte Reste $R^4$ in den obigen Formeln Ia, Ib, I-1, I-2, I-3 und I-4 (bzw. bevorzugte Reste $R^2$ in Formel I) sind Alkyl, Alkenyl, Alkoxy, Alkenyloxy, Haloalkoxy (vorzugsweise 2-Fluoralkoxy oder 2-Chloralkoxy), Alkoxycarbonyl und Haloalkanoyloxy (vorzugsweise 2-Fluoralkanoyloxy oder 2-Chloralkanoyloxy), insbesondere Alkyl, Alkenyl, Alkoxy, Alkenyloxy und 2-Chloralkoxy. Die Reste $R^4$ können geradkettig oder verzweigt sein und besitzen vorzugsweise höchstens 15, beispielsweise 2-15 Kohlenstoffatome und besonders bevorzugt etwa 3-10 Kohlenstoffatome. Beispiele bevorzugter Reste $R^4$ sind die eingangs genannten Gruppen. Besonders bevorzugte Reste $R^4$ sind Alkenyl, Alkenyloxy und insbesondere Alkyl und Alkoxy.

Die Verbindungen der Formel I können erfindungsgemäss dadurch hergestellt werden, dass man eine optisch aktive $\alpha$-Fluorcarbonsäure der allgemeinen Formel

$R^1$-C*HF-COOH III

und ein Cyclohexanol-Derivat der allgemeinen Formel

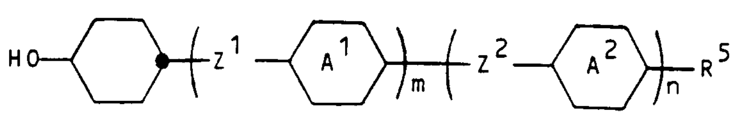

IV

worin m für die Zahl 1 steht und $R^5$ eine unsubstituierte oder mit Halogen substituierte Alkyl- oder Alkenylgruppe bezeichnet, in welcher gegebenenfalls eine Methylengruppe durch Sauerstoff ersetzt ist und/oder gegebenenfalls eine Methylengruppe durch eine Estergruppe -COO- oder -OOC- ersetzt ist; oder m für die Zahl 0 oder 1 steht und $R^5$ eine Gruppe der allgemeinen Formel

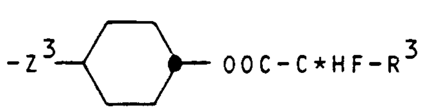

II

oder

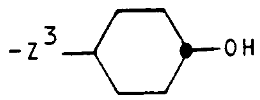

V

bezeichnet; $R^1$ und $R^3$ unabhängig voneinander eine Alkyl- oder Alkenylgruppe darstellen, in welcher gegebenenfalls eine Methylengruppe durch Sauerstoff ersetzt ist; $Z^1$, $Z^2$ und $Z^3$ unabhängig voneinander eine einfache Kovalenzbindung, $-CH_2O-$, $-OCH_2-$, $-CH_2CH_2-$, -COO- oder -OOC- bedeuten; n für die Zahl 0 oder 1 steht; die Ringe $A^1$ und $A^2$ unabhängig voneinander unsubstituiertes oder mit Halogen, Cyano und/oder Methyl substituiertes 1,4-Phenylen bedeuten, in welchem gegebenenfalls 1 oder 2 CH-Gruppen durch Stickstoff ersetzt sind; und $C^*$ ein chirales Kohlenstoffatom bezeichnet; mit der Massgabe, dass nicht zugleich $R^1$ eine Alkylgruppe, m und n die Zahl 1, $Z^1$ die Gruppe $-CH_2CH_2-$ oder $-CH_2O-$ und Ring $A^1$, $Z^2$, Ring $A^2$ und $R^5$ zusammen 4-(5-Alkyl-2-pyrimidinyl)phenyl bedeuten,
oder geeignete Derivate dieser Verbindungen verestert.

Die Veresterung der Verbindung der Formeln III und IV oder geeigneter Derivate dieser Verbindungen kann in an sich bekannter Weise nach den in Standardwerken (z.B. Houben-Weyl, Methoden der Organischen Chemie, Georg Thieme Verlag, Stuttgart) beschriebenen Methoden erfolgen.

Die Ausgangsmaterialien der Formel IV sind bekannte oder Analoge bekannter Verbindungen und können nach dem Fachmann bekannten Methoden hergestellt werden. Insbesondere wurden derartige Cyclohexanol-Derivate bereits zur Herstellung anderer Flüssigkristallkomponenten verwendet.

Die optisch aktiven α-Fluorcarbonsäuren der Formel III sind ebenfalls bekannte oder Analoge bekannter Verbindungen und können nach bekannten Methoden hergestellt werden. Beispielsweise können sie aus den racemischen α-Fluorcarbonsäuren durch übliche Racematspaltung, z.B. mit Ephedrin, erhalten werden. Ferner können racemische und optisch aktive α-Fluorcarbonsäuren beispielsweise hergestellt werden aus α-Hydroxycarbonsäureestern durch Umsetzung mit Diäthylaminoschwefeltrifluorid und anschliessende Hydrolyse der Estergruppe. Derartige Methoden und Ausgangsmaterialien sind ebenfalls bekannt aus Standardwerken, wie Houben-Weyl, Methoden der Organischen Chemie, Georg Thieme Verlag, Stuttgart.

α-Fluorcarbonsäuren sind zudem besonders leicht zugänglich durch Umsetzung von Aminosäuren, wie Norleucin, Isoleucin, Valin, Serin, Threonin und dergleichen, mit Natriumnitrit und Pyridin-hydrofluorid nach der in Helv. Chim. Acta 64, 2528 (1981) beschriebenen Methode (Falls Inversion am α-C-Atom gewünscht wird, kann z.B. zuerst in analoger Weise die α-Hydroxysäure hergestellt, dann diese mit Mesylchlorid zum Mesylat umgesetzt und das Mesylat mit Kaliumfluorid in die α-Fluorcarbonsäure übergeführt werden.) Im Falle von α-Aminosäuren ohne weitere funktionelle Gruppe, wie Leucin, Isoleucin und Valin, führt dies direkt zu den gewünschten α-Fluorcarbonsäuren. Im Falle von Hydroxy-α-aminosäuren, wie Serin und Threonin, werden auf diese Weise Hydroxy-α-fluorcarbonsäuren und durch anschliessende Veresterung der Carboxygruppe (z.B. mit Diazomethan) Hydroxy-α-fluorcarbonsäureester erhalten. Diese können beispielsweise durch Verätherung mit Alkylbromid oder Alkenylbromid und anschliessende Hydrolyse der Estergruppe in alkoxy- oder alkenyloxy-substituierte α-Fluorcarbonsäuren übergeführt werden. Ferner können die Hydroxy-α-fluorcarbonsäureester z.B. durch Oxidation mit Pyridiniumchlorochromat in formylsubstituierte α-Fluorcarbonsäureester übergeführt und diese durch Umsetzung mit Methoxymethyl-triphenylphosphoniumchlorid und anschliessende Hydrolyse des Enoläthers in homologe, formylsubstituierte α-Fluorcarbonsäureester übergeführt werden (diese Kettenverlängerungsreaktion kann gewünschtenfalls ein oder mehrmals wiederholt werden). Die formyl-substituierten α-Fluorcarbonsäureester können z.B. durch Reduktion mit Natriumborhydrid, Verätherung der Hydroxygruppe mit Alkylbromid oder Alkenylbromid und Hydrolyse der Estergruppe zu alkoxy- oder alkenyloxy-substituierten α-Fluorcarbonsäuren umgesetzt werden. Anderseits können die formylsubstituierten α-Fluorcarbonsäuren durch Wittig-Reaktion mit Alkyl-triphenylphosphoniumbromid, gewünschtenfalls katalytische Hydrierung der Doppelbindung und durch anschliessende Hydrolyse der Estergruppe in α-Fluorcarbonsäureester der Formel III übergeführt werden, worin $R^1$ Alkyl oder Alkenyl bedeutet.

Die Verbindungen der Formel I können als chirale Dotierstoffe in flüssigkristallinen Gemischen verwendet werden. Die Erfindung betrifft daher ebenfalls ein flüssigkristallines Gemisch mit mindestens 2 Komponenten, welches dadurch gekennzeichnet ist, dass mindestens eine Komponente eine optisch aktive Verbindung der Formel I ist. Zweckmässigerweise enthält das Gemisch ein Flüssigkristallmaterial mit nematischer, cholesterischer oder smektischer Phase und eine oder mehrer optisch aktive Verbindungen der Formel I.

Bevorzugt sind Gemische mit chiral getilteter smektischer Phase, insbesondere solche mit einer $S_C^*$ -Phase. diese Gemische enthalten vorzugsweise ein Flüssigkristallmaterial mit getilter smektischer Phase

(insbesondere mit einer smektisch C Phase) und eine oder mehrere optisch aktive Verbindungen der Formel I.

Der Anteil an Verbindungen der Formel I in den erfindungsgemässen Gemischen kann in breiten Grenzen variieren und beispielsweise etwa 0,5-40 Gew.-% betragen. Die bevorzugte Konzentration hängt im allgemeinen hauptsächlich von den gewünschten Werten der spontanen Polarisation und der Ganghöhe ab. Bevorzugt ist am allgemeinen ein Bereich von etwa 3-20 Gew.-% und besonders bevorzugt ein Bereich von etwa 5-15 Gew.-%.

Als weitere Komponenten kommen übliche Flüssigkristallmaterialien in Betracht. Vorzugsweise enthalten die erfindungsgemässen Gemische neben einer oder mehreren Verbindungen der Formel I eine oder mehrere der Verbindungen der folgenden allgemeinen Formeln

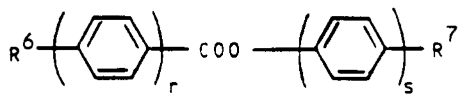
VI

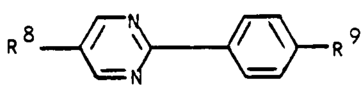
VII

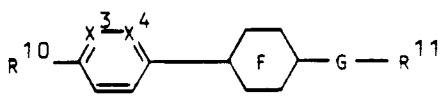
VIII

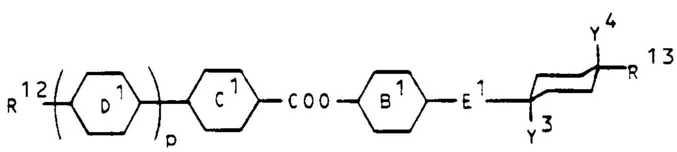
IX

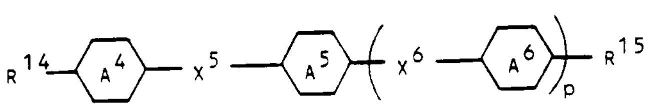
X

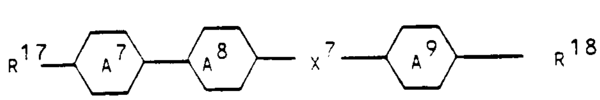
XI

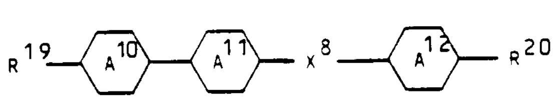
XII

worin $R^6$ und $R^7$ Alkyl, Alkoxy, Alkanoyl, Alkanoyloxy, Alkoxycarbonyl oder Alkoxycarbonyloxy mit bis zu 18 Kohlenstoffatomen bedeuten; r und s unabhängig voneinander 1 oder 2 bedeuten; $R^8$ und $R^9$ Alkyl oder Alkoxy mit 1-18 Kohlenstoffatomen darstellen; $X^3$ für CH und $X^4$ für N steht oder $X^3$ für N und $X^4$ für CH

steht; G eine einfache Kovalenzbindung, trans-1,4-Cyclohexylen, cis-4-Cyano-trans-1,4-cyclohexylen oder gegebenenfalls mit Halogen oder Methyl substituiertes 1,4-Phenylen bedeutet; Ring F trans-1,4-Cyclohexen, gegebenenfalls mit Halogen oder Methyl substituiertes 1,4-Phenylen oder, wenn G eine einfache Kovalenzbindung bedeutet, auch cis-4-Cyano-trans-1,4-cyclohexylen darstellt; $R^{10}$ und $R^{11}$ je eine gegebenenfalls halogensubstituierte Alkyl- oder Alkenylgruppe bezeichnen, in welcher gegebenenfalls eine oder zwei nicht benachbarte $CH_2$-Gruppen durch -O-, -COO- und/oder -OOC- ersetzt sind; p für die Zahl 0 oder 1 steht; $E^1$ eine einfache Kovalenzbindung, $-CH_2-CH_2-$, $-OCH_2-$, -COO- oder -OOC- bedeutet; die Ringe $B^1$, $C^1$ und $D^1$ gegebenenfalls mit Cyano, Halogen oder Niederalkyl substituiertes 1,4-Phenylen bezeichnen; $Y^3$ und $Y^4$ Wasserstoff bedeuten oder einer der Substituenten $Y^3$ und $Y^4$ auch Cyano bedeutet; $R^{12}$ und $R^{13}$ unabhängig voneinander gegebenenfalls halogensubstituiertes $C_1$-$C_{18}$-Alkyl oder gegebenenfalls halogensubstituiertes $C_2$-$C_{18}$-Alkenyl darstellen, in welchen gegebenenfalls eine oder zwei nicht benachbarte $CH_2$-Gruppen durch Sauerstoff ersetzt sind; $X^5$ eine einfache Kovalenzbindung, -COO- oder -OOC- und $X^5$ eine einfache Kovalenzbindung, -COO-, -OOC-, $CH_2CH_2$-, $-OCH_2$-oder $-CH_2O$- darstellen; die Ringe $A^4$, $A^5$ und $A^6$ unabhängig voneinander unsubstituiertes oder mit Cyano, Halogen oder Niederalkyl substituiertes 1,4-Phenylen oder einer der Ringe auch Pyrimidin-2,5-diyl oder Pyrazin-2,5-diyl bedeutet und/oder, wenn p für die Zahl 1 steht, einer der Ringe auch trans-1,4-Cyclohexylen oder trans-m-Dioxan-2,5-diyl bedeutet; $R^{14}$ eine gegebenenfalls halogensubstituierte Alkenylgruppe mit bis zu 18 Kohlenstoffatomen bedeutet, worin gegebenenfalls 1 oder 2 nicht benachbarte $CH_2$-Gruppen durch -O-, -CO-, -COO-oder -OOC- ersetzt sind und/oder gegebenenfalls eine C-C-Einfachbindung durch eine C-C-Doppelbindung ersetzt ist; $R^{15}$ eine gegebenenfalls halogensubstituierte Alkylgruppe mit bis zu 18 Kohlenstoffatomen bedeutet, worin gegebenenfalls 1 oder 2 nicht benachbarte $CH_2$-Gruppen durch -O-, -CO-, -COO- oder -OOC- ersetzt sind und/oder gegebenenfalls eine C-C-Einfachbindung durch eine C-C-Doppelbindung ersetzt ist; $X^7$ eine einfache Kovalenzbindung, -COO-, -OOC-, $-CH_2CH_2$-, $-OCH_2$- oder $-CH_2O$- bezeichnet; einer der Ringe $A^7$, $A^8$ und $A^9$ Pyrimidin-2,5-diyl darstellt, einer der Ringe $A^7$, $A^8$ und $A^9$ unsubstituiertes oder mit Cyano, Halogen oder Niederalkyl substituiertes 1,4-Phenylen darstellt und einer der Ringe $A^7$, $A^8$ und $A^9$ trans-1,4-Cyclohexylen oder unsubstituiertes oder mit Cyano, Halogen oder Niederalkyl substituiertes 1,4-Phenylen darstellt; und $R^{17}$ und $R^{18}$ unabhängig voneinander eine gegebenenfalls halogensubstituierte Alkylgruppe mit bis zu 18 Kohlenstoffatomen bedeuten, worin gegebenenfalls 1 oder 2 nicht benachbarte $CH_2$-Gruppen durch -O-, -CO-, -COO- und/oder -OOC- ersetzt sind; $X^5$ eine einfache Kovalenzbindung, -COO-, -OOC-, $-CH_2CH_2$-, $OCH_2$- oder $-CH_2O$- bezeichnet; einer der Ringe $A^{10}$, $A^{11}$ und $A^{12}$ trans-m-Dioxan-2,5-diyl darstellt, und die beiden andern der Ringe $A^{10}$, $A^{11}$ und $A^{12}$ unabhängig voneinander unsubstituiertes oder mit Cyano, Halogen oder Niederalkyl substituiertes 1,4-Phenylen darstellen; $R^{19}$ und $R^{20}$ unabhängig voneinander eine gegebenenfalls halogensubstituierte Alkylgruppe mit bis zu 18 Kohlenstoffatomen bedeuten, worin gegebenenfalls 1 oder 2 nicht benachbarte $CH_2$-Gruppen durch -O-, -CO-, -COO- und/oder -OOC- ersetzt sind.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Optische Antipoden besitzen jeweils die gleichen Phasenumwandlungstemperaturen und induzieren absolut gleiche Werte der spontanen Polarisation und der Verdrillung aber mit entgegengesetzten Vorzeichen. Die zur Charakterisierung der Phasenumwandlungen verwendeten Abklärungen stehen für folgende Bedeutungen:

| | |
|---|---|
| C | für kristallin |
| S | für smektisch |
| $S_A$, $S_B$ etc. | Für smektisch A, B etc. |
| $S_C^*$, $S_F^*$ etc. | für chiral smektisch C, F etc. |
| Ch | für cholesterisch |
| N | für nematisch |
| I | für isotrop. |

Beispiel 1

0,16 (S)-2-Fluorhexansäure (S-α-Fluorcapronsäure), 0,52 g trans-4-(4′-Decyl-4-biphenylyl)cyclohexanol und 0,02 g 4-(Dimethylamino)pyridin wurden in 50 ml Dichlormethan gelöst und die Lösung innert 10 Minuten unter Rühren portionenweise mit 0,35 g N,N′-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonat-Lösung und dann mit Wasser gewaschen, über Magnesiumsulfat getrocknet, filtriert und anschliessend eingeengt. Das erhaltene Rohprodukt wurde an Kieselgel mit Hexan/Aethylacetat (Vol. 9:1) chromatographisch gereinigt. Das trans-4-(4′-Decyl-4-biphenylyl)cyclohexyl-(S)-2-fluorhexanoat wurde aus Methanol umkristallisiert; Smp (C-S) -80 °C, Umwandlung S-S

-26 °C, Klp. (S-I) 144,5 °C.

Das als Ausgangsmaterial verwendete trans-4-(4′-Decyl-4-biphenylyl)cyclohexanol wurde wie folgt hergestellt:

(a) Eine Suspension von 10 g 4-(4′-Cyano-4-biphenylyl)cyclohexanon, 30 ml Diäthyläther und 270 ml Methanol wurde portionenweise mit 1,8 g Natriumborhydrid bei 0 °C versetzt. Nach 3 Stunden wurde das Reaktionsgemisch mit 25%iger Salzsäure sauer gestellt (pH 1-2). Das Reaktionsgemisch wurde dreimal mit je 50 ml Dichlormethan extrahiert. Die vereinigten organischen Phasen wurden zweimal mit je 500 ml Wasser gewaschen und anschliessend über Magnesiumsulfat getrocknet, filtriert und eingeengt. Umkristallisation aus Methanol ergab 3,5 g trans-4-(4′-Cyano-4-biphenylyl)cyclohexanol.

(b) Eine Lösung von 3,0 g trans-4-(4′-Cyano-4-biphenylyl)cyclohexanol, 1,1 ml 3,4-Dihydro-2H-pyran in 30 ml Dichlormethan wurde mit einer Lösung von 0,007 g Bistrimethylsilylsulfat in 2 ml Dichlormethan bei 0 °C versetzt. Das Reaktionsgemisch wurde noch 30 Minuten bei 0 °C gerührt und dann einmal mit Natriumcarbonat-Lösung und zweimal mit Wasser gewaschen und anschliessend über Magnesiumsulfat getrocknet, filtriert und eingeengt. Das Rohprodukt wurde an Kieselgel mit Toluol/Aethylacetat (Vol. 9:1) chromatographisch gereinigt. Umkristallisation aus Aethylacetat bei -20 °C ergab 1,75 g reinen trans-4-(4′-Cyano -4-biphenylyl)cyclohexyl-tetrahydropyranyläther.

(c) Eine Lösung von Nonylmagnesiumbromid (8,3 mMol) in 30 ml Diäthyläther wurde bei 0 °C mit einer Lösung von 1,5 g trans-4-(4′-Cyano -4-biphenylyl)cyclohexyl-tetrahydropyranyläther in 15 ml Tetrahydrofuran versetzt. Das Reaktionsgemisch wurde über Nacht bei leichtem Rückfluss erwärmt und dann mit verdünnter Natriumcarbonat-Lösung versetzt. Die organische Phase wurde abgetrennt und die wässrige Phase dreimal mit je 50 ml Diäthyläther nachextrahiert. Die vereinigten organischen Phasen wurden einmal mit verdünnter Natriumcarbonat-Lösung und zweimal mit Wasser gewaschen und dann über Magnesiumsulfat getrocknet, filtriert und eingeengt. Der Rückstand wurde an Kieselgel mit Toluol/Aethylacetat (Vol. 9:1) chromatographisch gereinigt. Der erhaltene trans-4-(4-Decanoyl -4-biphenylyl)cyclohexyl-tetrahydropyranyläther (1,8 g) wurde ohne weitere Reinigung in der nächsten Stufe eingesetzt.

(d) Ein Gemisch aus 1,8 g trans-4-(4′-Decanoyl -4-biphenylyl)cyclohexyl-tetrahydropyranyläther, 0,2 g Palladium/Kohle (5%), 20 ml Toluol und 2 ml Aethanol wurde während 16 Stunden unter Einleitung von Wasserstoff gerührt. Anschliessend wurde das anorganische Material abfiltriert und das Filtrat eingeengt. Der Rückstand wurde an Kieselgel mit Toluol/Aethylaceat (Vol. 9:1) chromatographisch gereinigt. Der erhaltene trans-4-(4′-Decyl -4-biphenylyl)cyclohexyl-tetrahydropyranyläther (1,2 g) wurde ohne weitere Reinigung in der nächsten Stufe eingesetzt.

(e) Eine Lösung von 1,2 g trans-4-(4′-Decyl -4-biphenylyl)cyclohexyl-tetrahydropyranyläther in 40 ml Methanol und 2 ml Diäthyläther wurde mit einer Lösung von 7,3 mg Bistrimethylsilylsulfat in 2 ml Dichlormethan versetzt. Das Reaktionsgemisch wude 2 Stunden bei Raumtemperatur gerührt und dann mit 0,2 ml Pyridin versetzt. Anschliessend wurde das Reaktionsgemisch eingeengt und dann der Rückstand in 50 ml Dichlormethan aufgenommen, zweimal mit Wasser gewaschen, über Magnesiumsulfat getrocknet, filtriert und nochmals eingeengt. Der Rückstand wurde an Kieselgel mit Toluol/Aethylacetat (Vol. 9:1) chromatographisch gereinigt. Umkristallisation aus Methanol ergab 0,5 g trans-4-(4′-Decyl-4-biphenylyl)cyclohexanol.

In analoger Weise können die folgenden Verbindungen hergestellt werden:

trans-4-(4′-Methyl-4-biphenylyl)cyclohexyl-(S)-2-fluorhexanoat;
trans-4-(4′-Aethyl-4-biphenylyl)cyclohexyl-(S)-2-fluorhexanoat;
trans-4-(4′-Propyl-4-biphenylyl)cyclohexyl-(S)-2-fluorhexanoat;
trans-4-(4′-Butyl-4-biphenylyl)cyclohexyl-(S)-2-fluorhexanoat;
trans-4-(4′-Pentyl-4-biphenylyl)cyclohexyl-(S)-2-fluorhexanoat;
trans-4-(4′-Hexyl-4-biphenylyl)cyclohexyl-(S)-2-fluorhexanoat;
trans-4-(4′-Heptyl-4-biphenylyl)cyclohexyl-(S)-2-fluorhexanoat;
trans-4-(4′-Octyl-4-biphenylyl)cyclohexyl-(S)-2-fluorhexanoat;
trans-4-(4′-Nonyl-4-biphenylyl)cyclohexyl-(S)-2-fluorhexanoat;
trans-4-(4′-Methoxy-4-biphenylyl)cyclohexyl-(S)-2-fluorhexanoat;
trans-4-(4′-Aethoxy-4-biphenylyl)cyclohexyl-(S)-2-fluorhexanoat;
trans-4-(4′-Propyloxy-4-biphenylyl)cyclohexyl-(S)-2-fluorhexanoat;
trans-4-(4′-Butyloxy-4-biphenylyl)cyclohexyl-(S)-2-fluorhexanoat;
trans-4-(4′-Pentyloxy-4-biphenylyl)cyclohexyl-(S)-2-fluorhexanoat;
trans-4-(4′-Hexyloxy-4-biphenylyl)cyclohexyl-(S)-2-fluorhexanoat;
trans-4-(4′-Octyloxy-4-biphenylyl)cyclohexyl-(S)-2-fluorhexanoat;
trans-4-(4′-Nonyloxy-4-biphenylyl)cyclohexyl-(S)-2-fluorhexanoat;

trans-4-(4′-Decyloxy-4-biphenylyl)cyclohexyl-(S)-2-fluorhexanoat;
trans-4-(3′-Fluor-4′-methoxy-4-biphenylyl)cyclohexyl-(S)-2-fluorhexanoat;
trans-4-(3′-Fluor-4′-äthoxy-4-biphenylyl)cyclohexyl-(S)-2-fluorhexanoat;
trans-4-(3′-Fluor-4′-propyloxy-4-biphenylyl)cyclohexyl-(S)-2-fluorhexanoat;
trans-4-(3′-Fluor-4′-butyloxy-4-biphenylyl)cyclohexyl-(S)-2-fluorhexanoat;
trans-4-(3′-Fluor-4′-pentyloxy-4-biphenylyl)cyclohexyl-(S)-2-fluorhexanoat;
trans-4-(3′-Fluor-4′-hexyloxy-4-biphenylyl)cyclohexyl-(S)-2-fluorhexanoat;
trans-4-(3′-Fluor-4′-heptyloxy-4-biphenylyl)cyclohexyl-(S)-2-fluorhexanoat;
trans-4-(3′-Fluor-4′-octyloxy-4-biphenylyl)cyclohexyl-(S)-2-fluorhexanoat;
trans-4-(3′-Fluor-4′-nonyloxy-4-biphenylyl)cyclohexyl-(S)-2-fluorhexanoat;
trans-4-(3′-Fluor-4′-decyloxy-4-biphenylyl)cyclohexyl-(S)-2-fluorhexanoat;
trans-4-(2′,3′-Difluor-4′-methoxy-4-biphenylyl)cyclohexyl-(S)-2-fluorhexanoat;
trans-4-(2′,3′-Difluor-4` -äthoxy-4-biphenylyl)cyclohexyl-(S)-2-fluorhexanoat;
trans-4-(2′,3′-Difluor-4` -propyloxy-4-biphenylyl)cyclohexyl-(S)-2-fluorhexanoat;
trans-4-(2′,3′-Difluor-4-butyloxy-4-biphenylyl)cyclohexyl-(S)-2-fluorhexanoat;
trans-4-(2′,3′-Difluor-4` -pentyloxy-4-biphenylyl)cyclohexyl-(S)-2-fluorhexanoat;
trans-4-(2′,3′-Difluor-4-hexyloxy-4-biphenyllyl)cyclolhexyl-(S)-2-fluorhexanoat;
trans-4-(2,′3′-Difluor-4` -heptyloxy-4-biphenylyl)cyclohexyl-(S)-2-fluorhexanoat;
trans-4-(2′.3′-Difluor-4` -otyloxy-4-biphenylyl)cyclohexyl-(S)-2-fluorhexanoat;
trans-4-(2′,3′-Difluor-4` -nonyloxy-4-biphenylyl)cyclohexyl-(S)-2-fluorhexanoat;
trans-4-(2′,3′-Difluor-4` -decyloxy-4-biphenyllyl)cyclohexyl-(S)-2-fluorhexanoat;
trans-4-(3′-Chlor-4′-methoxy-4-biphenylyl)cyclohexyl-(S)-2-fluorhexanoat;
trans-4-(3′-Chlor-4′-äthoxy-4-biphenylyl)cyclohexyl-(S)-2-fluorhexanoat;
trans-4-(3′-Chlor-4′-propyloxy-4-biphenylyl)cyclohexyl-(S)-2-fluorhexanoat;
trans-4-(3′-Chlor-4′-butyloxy-4-biphenylyl)cyclohexyl-(S)-2-fluorhexanoat;
trans-4-(3′-Chlor-4′-pentyloxy-4-biphenylyl)cyclohexyl-(S)-2-fluorhexanoat;
trans-4-(3′-Chlor-4′-hexyloxy-4-biphenylyl)cyclohexyl-(S)-2-fluorhexanoat;
trans-4-(3′-Chlor-4′-heptyloxy-4-biphenylyl)cyclohexyl-(S)-2-fluorhexanoat;
trans-4-(3′-Chlor-4′-octyloxy-4-biphenylyl)cyclohexyl-(S)-2-fluorhexanoat;
trans-4-(3′-Chlor-4′-nonyloxy-4-biphenylyl)cyclohexyl-(S)-2-fluorhexanoat;
trans-4-(3′-Chlor-4′-decyloxy-4-biphenylyl)cyclohexyl-(S)-2-fluorhexanoat;
trans-4-(3′-Brom-4′-methoxy-4-biphenylyl)cyclohexyl-(S)-2-fluorhexanoat;
trans-4-(3′-Brom-4′-äthoxy-4-biphenylyl)cyclohexyl-(S)-2-fluorhexanoat;
trans-4-(3′-Brom-4′-propyloxy-4-biphenylyl)cyclohexyl-(S)-2-fluorhexanoat;
trans-4-(3′-Brom-4′-butyloxy-4-biphenylyl)cyclohexyl-(S)-2-fluorhexanoat;
trans-4-(3′-Brom-4′-pentyloxy-4-biphenylyl)cyclohexyl-(S)-2-fluorhexanoat;
trans-4-(3′-Brom-4′-hexyloxy-4-biphenylyl)cyclohexyl-(S)-2-fluorhexanoat;
trans-4-(3′-Brom-4′-heptyloxy-4-biphenylyl)cyclohexyl-(S)-2-fluorhexanoat;
trans-4-(3′-Brom-4′-octyloxy-4-biphenylyl)cyclohexyl-(S)-2-fluorhexanoat;
trans-4-(3′-Brom-4′-nonyloxy-4-biphenylyl)cyclohexyl-(S)-2-fluorhexanoat;
trans-4-(3′-Brom-4′-decyloxy-4-biphenylyl)cyclohexyl-(S)-2-fluorhexanoat;
trans-4-(3′-Cyano-4′-methoxy-4-biphenylyl)cyclohexyl-(S)-2-fluorhexanoat;
trans-4-(3′-Cyano-4′-äthoxy-4-biphenylyl)cyclohexyl-(S)-2-fluorhexanoat;
trans-4-(3′-Cyano-4′-propyloxy-4-biphenylyl)cyclohexyl-(S)-2-fluorhexanoat;
trans-4-(3′-Cyano-4′-butyloxy-4-biphenylyl)cyclohexyl-(S)-2-fluorhexanoat;
trans-4-(3′-Cyano-4′-pentyloxy-4-biphenylyl)cyclohexyl-(S)-2-fluorhexanoat;
trans-4-(3′-Cyano-4′-hexyloxy-4-biphenylyl)cyclohexyl-(S)-2-fluorhexanoat;
trans-4-(3′-Cyano-4′-heptyloxy-4-biphenylyl)cyclohexyl-(S)-2-fluorhexanoat;
trans-4-(3′-Cyano-4′-octyloxy-4-biphenylyl)cyclohexyl-(S)-2-fluorhexanoat;
trans-4-(3′-Cyano-4′-nonyloxy-4-biphenylyl)cyclohexyl-(S)-2-fluorhexanoat;
trans-4-(3′-Cyano-4′-decyloxy-4-biphenylyl)cyclohexyl-(S)-2-fluorhexanoat;
trans-4-(2-[4′-Methyl-4-biphenylyl]äthyl)cyclohexyl-(S)-2-fluorhexanoat;
trans-4-(2-[4′-Aethyl-4-biphenylyl]äthyl)cyclohexyl-(S)-2-fluorhexanoat;
trans-4-(2-[4′-Propyl-4-biphenylyl]äthyl)cyclohexyl-(S)-2-fluorhexanoat;
trans-4-(2-[4′-Butyl-4-biphenylyl]äthyl)cyclohexyl-(S)-2-fluorhexanoat;
trans-4-(2-[4′-Pentyl-4-biphenylyl]äthyl)cyclohexyl-(S)-2-fluorhexanoat;
trans-4-(2-[4′-Hexyl-4-biphenylyl]äthyl)cyclohexyl-(S)-2-fluorhexanoat;
trans-4-(2-[4′-Heptyl-4-biphenylyl]äthyl)cyclohexyl-(S)-2-fluorhexanoat;

trans-4-(2-[4′-Octyl-4-biphenylyl]äthyl)cyclohexyl-(S)-2-fluorhexanoat;
trans-4-(2-[4′-Nonyl-4-biphenylyl]äthyl)cyclohexyl-(S)-2-fluorhexanoat;
trans-4-(2-[4′-Decyl-4-biphenylyl]äthyl)cyclohexyl-(S)-2-fluorhexanoat;
trans-4-(2-[4′-Methoxy-4-biphenylyl]äthyl)cyclohexyl-(S)-2-fluorhexanoat;
trans-4-(2-[4′-Aethoxy-4-biphenylyl]äthyl)cyclohexyl-(S)-2-fluorhexanoat;
trans-4-(2-[4′-Propyloxy-4-biphenylyl]äthyl)cyclohexyl-(S)-2-fluorhexanoat;
trans-4-(2-[4′-Butyloxy-4-biphenylyl]äthyl)cyclohexyl-(S)-2-fluorhexanoat;
trans-4-(2-[4′-Pentyloxy-4-biphenylyl]äthyl)cyclohexyl-(S)-2-fluorhexanoat;
trans-4-(2-[4′-Hexyloxy-4-biphenylyl]äthyl)cyclohexyl-(S)-2-fluorhexanoat;
trans-4-(2-[4′-Heptyloxy-4-biphenylyl]äthyl)cyclohexyl-(S)-2-fluorhexanoat;
trans-4-(2-[4′-Octyloxy-4-biphenylyl]äthyl)cyclohexyl-(S)-2-fluorhexanoat;
trans-4-(2-[4′-Nonyloxy-4-biphenylyl]äthyl)cyclohexyl-(S)-2-fluorhexanoat;
trans-4-(2-[4′-Decyloxy-4-biphenylyl]äthyl)cyclohexyl-(S)-2-fluorhexanoat;
trans-4-(2-[4′-Undecyloxy-4-biphenylyl]äthyl)cyclohexyl-(S)-2-fluorhexanoat;
trans-4-(2-[4′-Dodecyloxy-4-biphenylyl]äthyl)cyclohexyl-(S)-2-fluorhexanoat;
trans-4-(2-[3′-Fluor-4′-methoxy-4-biphenylyl]äthyl)cyclohexyl-(S)-2-fluorhexanoat;
trans-4-(2-[3′-Fluor-4′-äthoxy-4-biphenylyl]äthyl)cyclohexyl-(S)-2-fluorhexanoat;
trans-4-(2-[3′-Fluor-4′-propyloxy-4-biphenylyl]äthyl)cyclohexyl-(S)-2-fluorhexanoat;
trans-4-(2-[3′-Fluor-4′-butyloxy-4-biphenylyl]äthyl)cyclohexyl-(S)-2-fluorhexanoat;
trans-4-(2-[3′-Fluor-4′-pentyloxy-4-biphenylyl]äthyl)cyclohexyl-(S)-2-fluorhexanoat;
trans-4-(2-[3′-Fluor-4′-hexyloxy-4-biphenylyl]äthyl)cyclohexyl-(S)-2-fluorhexanoat;
trans-4-(2-[3′-Fluor-4′-heptyloxy-4-biphenylyl]äthyl)cyclohexyl-(S)-2-fluorhexanoat;
trans-4-(2-[3′-Fluor-4′-octyloxy-4-biphenylyl]äthyl)cyclohexyl-(S)-2-fluorhexanoat;
trans-4-(2-[3′-Fluor-4′-nonyloxy-4-biphenylyl]äthyl)cyclohexyl-(S)-2-fluorhexanoat;
trans-4-(2-[3′-Fluor-4′-decyloxy-4-biphenylyl]äthyl)cyclohexyl-(S)-2-fluorhexanoat;
trans-4-(2-[3′-Fluor-4′-undecyloxy-4-biphenylyl]äthyl)cyclohexyl-(S)-2-fluorhexanoat;
trans-4-(2-[3′-Fluor-4′-dodecyloxy-4-biphenylyl]äthyl)cyclohexyl-(S)-2-fluorhexanoat;
trans-4-(2-[3′-Chlor-4′-methoxy-4-biphenylyl]äthyl)cyclohexyl-(S)-2-fluorhexanoat;
trans-4-(2-[3′-Chlor-4′-äthoxy-4-biphenylyl]äthyl)cyclohexyl-(S)-2-fluorhexanoat;
trans-4-(2-[3′-Chlor-4′-propyloxy-4-biphenylyl]äthyl)cyclohexyl-(S)-2-fluorhexanoat;
trans-4-(2-[3′-Chlor-4′-butyloxy-4-biphenylyl]äthyl)cyclohexyl-(S)-2-fluorhexanoat;
trans-4-(2-[3′-Chlor-4′-pentyloxy-4-biphenylyl]äthyl)cyclohexyl-(S)-2-fluorhexanoat;
trans-4-(2-[3′-Chlor-4′-hexyloxy-4-biphenylyl]äthyl)cyclohexyl-(S)-2-fluorhexanoat;
trans-4-(2-[3′-Chlor-4′-heptyloxy-4-biphenylyl]äthyl)cyclohexyl-(S)-2-fluorhexanoat;
trans-4-(2-[3′-Chlor-4′-octyloxy-4-biphenylyl]äthyl)cyclohexyl-(S)-2-fluorhexanoat;
trans-4-(2-[3′-Chlor-4′-nonyloxy-4-biphenylyl]äthyl)cyclohexyl-(S)-2-fluorhexanoat;
trans-4-(2-[3′-Chlor-4′-decyloxy-4-biphenylyl]äthyl)cyclohexyl-(S)-2-fluorhexanoat;
trans-4-(2-[3′-Brom-4′-methoxy-4-biphenylyl]äthyl)cyclohexyl-(S)-2-fluorhexanoat;
trans-4-(2-[3′-Brom-4′-äthoxy-4-biphenylyl]äthyl)cyclohexyl-(S)-2-fluorhexanoat;
trans-4-(2-[3′-Brom-4′-propyloxy-4-biphenylyl]äthyl)cyclohexyl-(S)-2-fluorhexanoat;
trans-4-(2-[3′-Brom-4′-butyloxy-4-biphenylyl]äthyl)cyclohexyl-(S)-2-fluorhexanoat;
trans-4-(2-[3′-Brom-4′-pentyloxy-4-biphenylyl]äthyl)cyclohexyl-(S)-2-fluorhexanoat;
trans-4-(2-[3′-Brom-4′-hexyloxy-4-biphenylyl]äthyl)cyclohexyl-(S)-2-fluorhexanoat;
trans-4-(2-[3′-Brom-4′-heptyloxy-4-biphenylyl]äthyl)cyclohexyl-(S)-2-fluorhexanoat;
trans-4-(2-[3′-Brom-4′-octyloxy-4-biphenylyl]äthyl)cyclohexyl-(S)-2-fluorhexanoat;
trans-4-(2-[3′-Brom-4′-nonyloxy-4-biphenylyl]äthyl)cyclohexyl-(S)-2-fluorhexanoat;
trans-4-(2-[3′-Brom-4′-decyloxy-4-biphenylyl]äthyl)cyclohexyl-(S)-2-fluorhexanoat;
trans-4-(2-[3′-Cyano-4′-methoxy-4-biphenylyl]äthyl)cyclohexyl-(S)-2-fluorhexanoat;
trans-4-(2-[3′-Cyano-4′-äthoxy-4-biphenylyl]äthyl)cyclohexyl-(S)-2-fluorhexanoat;
trans-4-(2-[3′-Cyano-4′-propyloxy-4-biphenylyl]äthyl)cyclohexyl-(S)-2-fluorhexanoat;
trans-4-(2-[3′-Cyano-4′-butyloxy-4-biphenylyl]äthyl)cyclohexyl-(S)-2-fluorhexanoat;
trans-4-(2-[3′-Cyano-4′-pentyloxy-4-biphenylyl]äthyl)cyclohexyl-(S)-2-fluorhexanoat;
trans-4-(2-[3′-Cyano-4′-hexyloxy-4-biphenylyl]äthyl)cyclohexyl-(S)-2-fluorhexanoat;
trans-4-(2-[3′-Cyano-4′-heptyloxy-4-biphenylyl]äthyl)cyclohexyl-(S)-2-fluorhexanoat;
trans-4-(2-[3′-Cyano-4′-octyloxy-4-biphenylyl]äthyl)cyclohexyl-(S)-2-fluorhexanoat;
trans-4-(2-[3′-Cyano-4′-nonyloxy-4-biphenylyl]äthyl)cyclohexyl-(S)-2-fluorhexanoat;
trans-4-(2-[3′-Cyano-4′-decyloxy-4-biphenylyl]äthyl)cyclohexyl-(S)-2-fluorhexanoat;
trans-4-[2-(4′-[(S)-2-Octyloxy]-4-biphenylyl)äthyl)cyclohexyl-(S)-2-fluorhexanoat;

trans-4-[2-(3′-Fluor-4′-[(S)-2-octyloxy]-4-biphenylyl)äthyl]cyclohexyl-(S)-2-fluorhexanoat;
trans-4-[2-(3′-Chlor-4′-[(S)-2-octyloxy]-4-biphenylyl)äthyl]cyclohexyl-(S)-2-fluorhexanoat;
trans-4-[2-(3′-Brom-4′-[(S)-2-octyloxy]-4-biphenylyl)äthyl]cyclohexyl-(S)-2-fluorhexanoat;
trans-4-[2-(3′-Cyano-4′-[(S)-2-octyloxy]-4-biphenylyl)äthyl]cyclohexyl-(S)-2-fluorhexanoat;
trans-4-[2-(4′-[(R)-2-Octyloxy]-4-biphenylyl)äthyl]cyclohexyl-(S)-2-fluorhexanoat;
trans-4-[2-(3′-Fluor-4′-[(R)-2-octyloxy]-4-biphenylyl)äthyl]cyclohexyl-(S)-2-fluorhexanoat;
trans-4-[2-(3′-Chlor-4′-[(R)-2-octyloxly]-4-biphenyl)äthyl]cyclohexyl-(S)-2-fluorhexanoat;
trans-4-[2-(3′-Brom-4′-[(R)-2-octyloxy]-4-biphenylyl)äthyl]cyclohexyl-(S)-2-fluorhexanoat;
trans-4-[2-(3′-Cyano-4′-[(R)-2-octyloxy]-4-biphenylyl)äthyl]cyclohexyl-(S)-2-fluorhexanoat;
trans-4-[2-(4′-[(S)-2-Chlor-1-propyloxy]-4-biphenylyl)äthyl]cyclohexyl-(S)-2-fluorhexanoat;
trans-4-[2-(4′-[(S)-2-Chlor-1-propyloxy]-4-biphenylyl)äthyl]cyclohexyl-(R)-2-fluorhexanoat;
trans-4-[2-(4′-[(R)-2-Chlor-1-propyloxy]-4-biphenylyl)äthyl]cyclohexyl-(S)-2-fluorhexanoat;
trans-4-[2-(4′-[(R)-2-Chlor-1-propyloxy]-4-biphenylyl)äthyl]cyclohexyl-(R)-2-fluorhexanoat;
trans-4-(4-Methoxyphenyl)cyclohexyl)-(S)-2-fluorhexanoat;
trans-4-(4-Aethoxyphenyl)cyclohexyl-(S)-2-fluorhexanoat;
trans-4-(4-Propyloxyphenyl)cyclohexyl-(S)-2-fluorhexanoat;
trans-4-(4-Butyloxyphenyl)cyclohexyl-(S)-2-fluorhexanoat;
trans-4-(4-Pentyloxyphenyl)cyclohexyl-(S)-2-fluorhexanoat;
trans-4-(4-Hexyloxyphenyl)cyclohexyl-(S)-2-fluorhexanoat;
trans-4-(4-Heptyloxylphenyl)cyclohexyl-(S)-2-fluorhexanoat;
trans-4-(4-Otyloxyphenyl)cyclohexyl-(S)-2-fluorhexanoat;
trans-4-(4-Nonyloxyphenyl)cyclohexyl-(S)-2-fluorhexanoat;
trans-4-(4-Decylocyphenyl)cyclohexyl-(S)2-fluorhexanoat Smp.(C-I)42,5°. Klp.($S_B$-I)41°C;
trans-4-Undecyloxylphenyl)cyclohexyl-(S)-2-fluorhexanoat;
trans-4-Dodecyloxyphenyl)cyclohexyl-(S)-2-fluorhexanoat;

In analoger Weise können durch Versterung von trans-4-[4-(5-Alkyl-2-pyrimidinyl)phenyl]cyclohexanolen die nachstehend aufgeführten Verbindungen hergestellt werden; die Ausgangsdmaterialien wurden durch Reduktion von 4-(4-Cyanophenyl)cyclohexanon mit Natriumborhydrid und weitere Umsetzung des trans-4-(4-Cyanophenyl)cyclohexanols in analoger Weise zu Z. Naturforschung 34b, 1535(1979) erhalten:

trans-4-[4-(5-Methyl-2-pyrimidinyl)phenyl]cyclohexyl-(S)-2-fluorhexanoat;
trans-4-[4-(5-Aethyl-2-pyrimidinyl)phenyl]cyclohexyl-(S)-2-fluorhexanoat;
trans-4-[4-(5-Propyl-2-pyrimidinyl)phenyl]cyclohexyl-(S)-2-fluorhexanoat;
trans-4-[4-(5-Butyl-2-pyrimidinyl)phenyl]cyclohexyl-(S)-2-fluorhexanoat;
trans-4-[4-(5-Pentyl-2-pyrimidinyl)phenyl]cyclohexyl-(S)-2-fluorhexanoat;

trans-4-[4-(5-Hexyl-2-pyrimidinyl)phenyl]cyclohexyl-(S)-2-fluorhexanoat, Smp. (C-$S_B$)34°C, Umwandlung ($S_B$-$S_A$)98°C, Klp. ($S_A$-I)137°C;

trans-4-[4-(5-Heptyl-2-pyrimidinyl)phenyl]cyclohexyl-(S)-2-2-fluorhexanoat, Smp. (C-$S_B$)53°C, Umwandlung ($S_B$-$S_A$)101°C, Klp. ($S_A$-I)143°C;

trans-4-[4-(5-Octyl-2-pyrimidinyl)phenyl]cyclohexyl-(S)-2-fluorhexanoat, Smp. (C-$S_B$)41°C, Umwandlung ($S_B$-$S_A$)109°C, Klp. ($S_A$-I)143°C;

trans-4-[4-(5-Nonyl-2-pyrimidinyl)phenyl]cyclohexyl-(S)-2-fluorhexanoat; Smp. (C-$S_B$)49°C, Umwandlung ($S_B$-$S_A$)113°, Klp. ($S_A$-I) 145°C,

trans-4-[4-(5-Decyl-2-pyrimidinyl)phenyl]cyclohexyl-(S)-2-fluorhexanoat; Smp. C-$S_B$)48°C, Umwandlung ($S_B$-$S_A$)116°C. Klp ($S_A$-I) 145°C;

trans-4-[4-(5-Methyl-2-pyrimidinyl)phenyl]cyclohexyl-(2S,3S)-2-fluor-3-methylpentanoat;
trans-4-[4-(5-Aethyl-2-pyrimidinyl)phenyl]cyclohexyl-(2S,3S)-2-fluor-3-methylpentanoat;
trans-4-[4-(5-Propyl-2-pyrimidinyl)phenyl]cyclohexyl-(2S,3S)-2-fluor-3-methylpentanoat;
trans-4-[4-(5-Butyl-2-pyrimidinyl)phenyl]cyclohexyl-(2S,3S)-2-fluor-3-methylpentanoat;
trans-4-[4-(5-Pentyl-2-pyrimidinyl)phenyl]cyclohexyl-(2S,3S)-2-fluor-3-methylpentanoat;
trans-4-[4-(5-Hexyl-2-pyrimidinyl)phenyl]cyclohexyl-(2S,3S)-2-fluor-3-methylpentanoat;
trans-4-[4-(5-Heptyl-2-pyrimidinyl)phenyl]cyclohexyl-(2S,3S)-2-fluor-3-methylpentanoat;
trans-4-[4-(5-Octyl-2-pyrimidinyl)phenyl]cyclohexyl-(2S,3S)-2-fluor-3-methylpentanoat;
trans-4-[4-(5-Nonyl-2-pyrimidinyl)phenyl]cyclohexyl-(2S,3S)-2-fluor-3-methylpentanoat;
trans-4-[4-(5-Decyl)-2-pyrimidinyl)phenyl]cyclohexyl-(2S,3S)-2-fluor-3-methylpentanoat.

In analoger Weise können durch Veresterung von trans-4-(4′-Alkoxy-2′,3′-difluor-4-biphenylyl)-cyclohexanolen die nachstehend aufgeführten Verbindungen hergestellt werden; die Ausgangsmaterialien wurden nach bekannten Methoden erhalten durch Verätherung von 2,3-Difluorphenol mit Alkylbromid, anschliessende Umsetzung mit Butyllithium und dem Monoketal von Bicyclohexyl-4,4`-dion mit Aethylenglykol, Dehydratisierung mit Toluolsulfonsäure, Umsetzung mit 2,3-Dichlor-5,6-dicyan-1,4-benzochinon, Hydrolyse der Ketalgruppe und Reduktion des erhaltenen Ketons mit Natriumborhydrid zum Alkohol.

trans-4-(4′-Methoxy-2′,3′-difluor-4-biphenylyl)cyclohexyl-(S)-2-fluorhexanoat;
trans-4-(4′-Aethoxy -2′,3′-difluor-4-biphenylyl)cyclohexyl-(S)-2-fluorhexanoat;
trans-4-(4′-Propyloxy-2′,3′-difluor-4-biphenylyl)cyclohexyl-(S)-2-fluorhexanoat;
trans-4-(4′-Butyloxy-2′,3′-difluor-4-biphenyl)cyclohexyl-(S)-2-fluorhexanoat;
trans-4-(4′-Pentyloxy-2′,3′-difluor-4-biphenylyl)cyclohexyl-(S)-2-fluorhexanoat;
trans-4-(4′-Hexyloxy-2′,3′-difluor-4-biphenylyl)cyclohexyl-(S)-2-fluorhexanoat;
trans-4-(4′-Heptyloxy-2′,3′-difluor-4-biphenylyl)cyclohexyl-(S)-2-fluorhexanoat;
trans-4-(4′-Octyloxy-2′,3′-difluor-4-biphenylyl)cyclohexyl-(S)-2-fluorhexanoat;
trans-4-(4′-Nonyloxy-2′,3′-difluor-4-biphenylyl)cyclohexyl-(S)-2-fluorhexanoat;
trans-4-(4′-Decyloxy-2′,3′-difluor-4-biphenylyl)cyclohexyl-(S)-2-fluorhexanoat;
trans-4-(4′-Methoxy-2′,3′-difluor-4-biphenylyl)cyclohexyl-(2S,3S)-2-fluor-3-methylpentanoat;
trans-4-(4′-Aethoxy-2′,3′-difluor-4-biphenylyl)cyclohexyl-(2S,3S)-2-fluor-3-methylpentanoat;
trans-4-(4′-Propyloxy-2′,3′-difluor-4-biphenylyl)cyclohexyl-(2S,3S)-2-fluor-3-methylpentanoat;
trans-4-(4′-Butyloxy-2′,3′-difluor-4-biphenylyl)cyclohexyl-(2S,3S)-2-fluor-3-methylpentanoat;
trans-4-(4′-Pentyloxy-2′,3′-difluor-4-biphenylyl)cyclohexyl-(25,35)-2-fluor-3-methylpentanoat;
trans-4-(4′-Hexyloxy-2′,3′-difluor-4-biphenylyl)cyclohexyl-(2S,3S)-2-fluor-3-methylpentanoat;
trans-4-(4′-Heptyloxy-2′,3′-difluor-4-biphenylyl)cyclohexyl-(2S,3S)-2-fluor-3-methylpentanoat;
trans-4-(4′-Octyloxy)-2′,3′-difluor-4-biphenylyl)cyclohexyl-(2S,3S)-2-fluor-3-methylpentanoat;
trans-4-(4′-Nonyloxy-2′,3′-difluor-4-biphenylyl)cyclohexyl-(2S,3S)-2-fluor-3-methylpentanoat;
trans-4-(4′-Decyloxy-2′,3′-difluor-4-biphenylyl)cyclohexyl-(2S,3S)-2-fluor-3-methylpentanoat

Beispiel 2

0,2 g (S)-2-Fluorhexansäure (S-α-Fluorcapronsäure), 0,5 g trans-4-([2-Brom-4-(5-decyl-2-pyrimidinyl)-phenoxy]- methyl)cyclohexanol, 0,35 g N,N′-Dicyclohexylcarbodiimid, 0,02 g 4-(Dimethylamino)pyridin und 50 ml Dichlormethan wurden in analoger Weise zu Beispiel 1 umgesetzt. Dies ergab 0,4 g trans-4-([2-Brom-4-(5-decyl-2-pyrimidinyl)phenoxy]-methyl)cyclohexyl-(S)-2-fluorhexanoat.

Das als Ausgangsmaterial verwendete trans-4-([2-Brom-4-(5-decyl -2-pyrimidinyl)phenoxy]methyl)-cyclohexanol wurde wie folgt hergestellt:

(a) Eine Lösung von 5 g 4-(5-Decyl-2-pyrimidinyl)phenol in 50 ml Dichlormethan wurde bei 0°C unter Argonbegasung mit einer Lösung von 2,6 g Brom in 50 ml Dichlormethan versetzt. Das Reaktionsgemisch wurde noch 2 Stunden gerührt und dann mit Wasser versetzt. Die organische Phase wurde abgetrennt und die wässrige Phase dreimal mit je 50 ml Dichlormethan nachextrahiert. Die vereinigten organischen Phasen wurden mit Wasser, Natriumhydrogencarbonat-Lösung und nochmals mit Wasser gewaschen, über Magnesiumsulfat getrocknet, filtriert und anschliessend eingeengt. Der Rückstand wurde an Kieselgel mit Toluol/Aethylacetat (Vol. 4:1) chromatographisch gereinigt. Umkristallisation aus Hexan ergab 4,8 g 2-Brom-4-(5-decyl-2-pyrimidinyl)phenol.

(b) Ein Gemisch von 4,8 g 2-Brom-4-(5-decyl-2-pyrimidinyl)phenol, 3,5 g (trans-4-Hydroxycyclohexyl)-methyl-tosylat, 6,8 g Kaliumcarbonat und 100 ml Butanon wurde 2 Tage unter leichtem Rückfluss erhitzt. Anschliessend wurde das abgekühlte Reaktionsgemisch auf Wasser gegossen und dreimal mit je 50 ml Dichlormethan extrahiert. Die vereinigten organischen Phasen wurden mit Wasser gewaschen, über Magnesiumsulfat getrocknet, filtriert und anschliessend eingeengt. Der Rückstand wurde an Kieselgel mit Toluol/Aethylacetat (Vol. 4:1) chromatographisch gereinigt. Dies ergab 4,5 g trans-4-([2-Brom-4-(5-decyl -2-pyrimidinyl)phenoxy]methyl)cyclohexanol.

In analoger Weise können folgende Verbindungen hergestellt werden:

trans-4-([2-Fluor-4-(5-methyl-2-pyrimidinyl)phenoxy]methyl)cyclohexyl-(S)-2-fluorhexanoat;
trans-4-([2-Fluor-4-(5-äthyl-2-pyrimidinyl)phenoxy]methyl)cyclohexyl-(S)-2-fluorhexanoat;
trans-4-([2-Fluor-4-(5-propyl-2-pyrimidinyl)phenoxy]methyl)cyclohexyl-(S)-2-fluorhexanoat;
trans-4-([2-Fluor-4-(5-butyl-2-pyrimidinyl)phenoxy]methyl)cyclohexyl-(S)-2-fluorhexanoat;
trans-4-([2-Fluor-4-(5-pentyl-2-pyrimidinyl)phenoxy]methyl)cyclohexyl-(S)-2-fluorhexanoat;
trans-4-([2-Fluor-4-(5-hexyl-2-pyrimidinyl)phenoxy]methyl)cyclohexyl-(S)-2-fluorhexanoat;
trans-4-([2-Fluor-4-(5-heptyl-2-pyrimidinyl)phenoxy]methyl)cyclohexyl-(S)-2-fluorhexanoat;
trans-4-([2-Fluor-4-(5-octyl-2-pyrimidinyl)phenoxy]methyl)cyclohexyl-(S)-2-fluorhexanoat;
trans-4-([2-Fluor-4-(5-nonyl-2-pyrimidinyl)phenoxy]methyl)cyclohexyl-(S)-2-fluorhexanoat;
trans-4-([2-Fluor-4-(5-decyl-2-pyrimidinyl)phenoxy]methyl)cyclohexyl-(S)-2-fluorhexanoat;
trans-4-([2-Chlor-4-(5-methyl-2-pyrimidinyl)phenoxy]methyl)cyclohexyl-(S)-2-fluorhexanoat;

trans-4-([2-Chlor-4-(5-äthyl-2-pyrimidinyl)phenoxy]methyl)cyclohexyl-(S)-2-fluorhexanoat;
trans-4-([2-Chlor-4-(5-propyl-2-pyrimidinyl)phenoxy]methyl)cyclohexyl-(S)-2-fluorhexanoat;
trans-4-([2-Chlor-4-(5-butyl-2-pyrimidinyl)phenoxy]methyl)cyclohexyl-(S)-2-fluorhexanoat;
trans-4-([2-Chlor-4-(5-pentyl-2-pyrimidinyl)phenoxy]methyl)cyclohexyl-(S)-2-fluorhexanoat;
trans-4-([2-Chlor-4-(5-hexyl-2-pyrimidinyl)phenoxy]methyl)cyclohexyl-(S)-2-fluorhexanoat;
trans-4-([2-Chlor-4-(5-heptyl-2-pyrimidinyl)phenoxy]methyl)cyclohexyl-(S)-2-fluorhexanoat;
trans-4-([2-Chlor-4-(5-octyl-2-pyrimidinyl)phenoxy]methyl)cyclohexyl-(S)-2-fluorhexanoat;
trans-4-([2-Chlor-4-(5-nonyl-2-pyrimidinyl)phenoxy]methyl)cyclohexyl-(S)-2-fluorhexanoat;
trans-4-([2-Chlor-4-(5-decyl-2-pyrimidinyl)phenoxy]methyl)cyclohexyl-(S)-2-fluorhexanoat;
trans-4-([2-Brom-4-(5-methyl-2-pyrimidinyl)phenoxy]methyl)cyclohexyl-(S)-2-fluorhexanoat;
trans-4-([2-Brom-4-(5-äthyl-2-pyrimidinyl)phenoxy]methyl)cyclohexyl-(S)-2-fluorhexanoat;
trans-4-([2-Brom-4-(5-propyl-2-pyrimidinyl)phenoxy]methyl)cyclohexyl-(S)-2-fluorhexanoat;
trans-4-([2-Brom-4-(5-butyl-2-pyrimidinyl)phenoxy]methyl)cyclohexyl-(S)-2-fluorhexanoat;
trans-4-([2-Brom-4-(5-pentyl-2-pyrimidinyl)phenoxy]methyl)cyclohexyl-(S)-2-fluorhexanoat;
trans-4-([2-Brom-4-(5-hexyl-2-pyrimidinyl)phenoxy]methyl)cyclohexyl-(S)-2-fluorhexanoat;
trans-4-([2-Brom-4-(5-heptyl-2-pyrimidinyl)phenoxy]methyl)cyclohexyl-(S)-2-fluorhexanoat;
trans-4-([2-Brom-4-(5-octyl-2-pyrimidinyl)phenoxy]methyl)cyclohexyl-(S)-2-fluorhexanoat;
trans-4-([2-Brom-4-(5-nonyl-2-pyrimidinyl)phenoxy]methyl)cyclohexyl-(S)-2-fluorhexanoat;
trans-4-([2-Cyano-4-(5-methyl-2-pyrimidinyl)phenoxy]methyl)cyclohexyl-(S)-2-fluorhexanoat;
trans-4-([2-Cyano-4-(5-äthyl-2-pyrimidinyl)phenoxy]methyl)cyclohexyl-(S)-2-fluorhexanoat;
trans-4-([2-Cyano-4-(5-propyl-2-pyrimidinyl)phenoxy]methyl)cyclohexyl-(S)-2-fluorhexanoat;
trans-4-([2-Cyano-4-(5-butyl-2-pyrimidinyl)phenoxy]methyl)cyclohexyl-(S)-2-fluorhexanoat;
trans-4-([2-Cyano-4-(5-pentyl-2-pyrimidinyl)phenoxy]methyl)cyclohexyl-(S)-2-fluorhexanoat;
trans-4-([2-Cyano-4-(5-hexyl-2-pyrimidinyl)phenoxy]methyl)cyclohexyl-(S)-2-fluorhexanoat;
trans-4-([2-Cyano-4-(5-heptyl-2-pyrimidinyl)phenoxy]methyl)cyclohexyl-(S)-2-fluorhexanoat;
trans-4-([2-Cyano-4-(5-octyl-2-pyrimidinyl)phenoxy]methyl)cyclohexyl-(S)-2-fluorhexanoat;
trans-4-([2-Cyano-4-(5-nonyl-2-pyrimidinyl)phenoxy]methyl)cyclohexyl-(S)-2-fluorhexanoat;
trans-4-([2-Cyano-4-(5-decyl-2-pyrimidinyl)phenoxy]methyl)cyclohexyl-(S)-2-fluorhexanoat;
trans-4-([4′-Methyl-4-biphenylyloxy]methyl)cyclohexyl-(S)-2-fluorhexanoat;
trans-4-([4′-Aethyl-4-biphenylyloxy]methyl)cyclohexyl-(S)-2-fluorhexanoat;
trans-4-([4′-Propyl-4-biphenylyloxy]methyl)cyclohexyl-(S)-2-fluorhexanoat;
trans-4-([4′-Butyl-4-biphenylyloxy]methyl)cyclohexyl-(S)-2-fluorhexanoat;
trans-4-([4′-Pentyl-4-biphenylyloxy]methyl)cyclohexyl-(S)-2-fluorhexanoat;
trans-4-([4′-Hexyl-4-biphenylyloxy]methyl)cyclohexyl-(S)-2-fluorhexanoat;
trans-4-([4′-Heptyl-4-biphenylyloxy]methyl)cyclohexyl-(S)-2-fluorhexanoat;
trans-4-([4′-Octyl-4-biphenylyloxy]methyl)cyclohexyl-(S)-2-fluorhexanoat;
trans-4-([4′-Nonyl-4-biphenylyloxy]methyl)cyclohexyl-(S)-2-fluorhexanoat;
trans-4-([4′-Decyl-4-biphenylyloxy]methyl)cyclohexyl-(S)-2-fluorhexanoat;
trans-4-([4′-Undecyl-4-biphenylyloxy]methyl)cyclohexyl-(S)-2-fluorhexanoat;
trans-4-([4′-Dodecyl-4-biphenylyloxy]methyl)cyclohexyl-(S)-2-fluorhexanoat;
trans-4-([4′-Methoxy-4-biphenylyloxy]methyl)cyclohexyl-(S)-2-fluorhexanoat;
trans-4-([4′-Aethoxy-4-biphenylyloxy]methyl)cyclohexyl-(S)-2-fluorhexanoat;
trans-4-([4′-Propyloxy-4-biphenylyloxy]methyl)cyclohexyl-(S)-2-fluorhexanoat;
trans-4-([4′-Butyloxy-4-biphenylyloxy]methyl)cyclohexyl-(S)-2-fluorhexanoat;
trans-4-([4′-Pentyloxy-4-biphenylyloxy]methyl)cyclohexyl-(S)-2-fluorhexanoat;
trans-4-([4′-Hexyloxy-4-biphenylyloxy]methyl)cyclohexyl-(S)-2-fluorhexanoat;
trans-4-([4′-Heptyloxy-4-biphenylyloxy]methyl)cyclohexyl-(S)-2-fluorhexanoat;
trans-4-([4′-Octyloxy-4-biphenylyloxy]methyl)cyclohexyl-(S)-2-fluorhexanoat;
trans-4-([4′-Nonyloxy-4-biphenylyloxy]methyl)cyclohexyl-(S)-2-fluorhexanoat;
trans-4-([4′-Decyloxy-4-biphenylyloxy]methyl)cyclohexyl-(S)-2-fluorhexanoat;
trans-4-([3′-Fluor-4′-octyloxy-4-biphenylyloxy]methyl)cyclohexyl-(S)-2-fluorhexanoat;
trans-4-([3′-Chlor-4′-octyloxy-4-biphenylyloxy]methyl)cyclohexyl-(S)-2-fluorhexanoat;
trans-4-([3′-Brom-4′-octyloxy-4-biphenylyloxy]methyl)cyclohexyl-(S)-2-fluorhexanoat;
trans-4-([3′-Cyano-4′-octyloxy-4-biphenylyloxy]methyl)cyclohexyl-(S)-2-fluorhexanoat;
trans-4-([3′-Fluor-4′-octyloxy-4-biphenylyloxy]methyl)cyclohexyl-(R)-2-fluorhexanoat;
trans-4-([3′-Chlor-4′-octyloxy-4-biphenylyloxy]methyl)cyclohexyl-(R)-2-fluorhexanoat;
trans-4-([3′-Brom-4′-octyloxy-4-biphenylyloxy]methyl)cyclohexyl-(R)-2-fluorhexanoat;
trans-4-([3′-Cyano-4′-octyloxy-4-biphenylyloxy]methyl)cyclohexyl-(R)-2-fluorhexanoat;

trans-4-([4′-[(S)-2-Octyloxy]-4-biphenylyloxy]methyl)cyclohexyl-(S)-2-fluorhexanoat;
trans-4-([4′-[(S)-2-Octyloxy]-4-biphenylyloxy]methyl)cyclohexyl-(R)-2-fluorhexanoat;
trans-4-([4-(5-Methyl-2-pyridinyl)phenoxy]methyl)cyclohexyl-(S)-2-fluorhexanoat;
trans-4-([4-(5-Aethyl-2-pyridinyl)phenoxy]methyl)cyclohexyl-(S)-2-fluorhexanoat;
trans-4-([4-(5-Propyl-2-pyridinyl)phenoxy]methyl)cyclohexyl-(S)-2-fluorhexanoat;
trans-4-([4-(5-Butyl-2-pyridinyl)phenoxy]methyl)cyclohexyl-(S)-2-fluorhexanoat;
trans-4-([4-(5-Pentyl-2-pyridinyl)phenoxy]methyl)cyclohexyl-(S)-2-fluorhexanoat;
trans-4-([4-(5-Hexyl-2-pyridinyl)phenoxy]methyl)cyclohexyl-(S)-2-fluorhexanoat;
trans-4-([4-(5-Heptyl-2-pyridinyl)phenoxy]methyl)cyclohexyl-(S)-2-fluorhexanoat;
trans-4-([4-(5-Octyl-2-pyridinyl)phenoxy]methyl)cyclohexyl-(S)-2-fluorhexanoat;
trans-4-([4-(5-Nonyl-2-pyridinyl)phenoxy]methyl)cyclohexyl-(S)-2-fluorhexanoat;
trans-4-([4-(5-Decyl-2-pyridinyl)phenoxy]methyl)cyclohexyl-(S)-2-fluorhexanoat;
trans-4-([2-Fluor-4-(5-decyl-2-pyridinyl)phenoxy]methyl)cyclohexyl-(S)-2-fluorhexanoat;
trans-4-([2-Chlor-4-(5-decyl-2-pyridinyl)phenoxy]methyl)cyclohexyl-(S)-2-fluorhexanoat;
trans-4-([2-Brom-4-(5-decyl-2-pyridinyl)phenoxy]methyl)cyclohexyl-(S)-2-fluorhexanoat;
trans-4-([2-Cyano-4-(5-decyl-2-pyridinyl)phenoxy]methyl)cyclohexyl-(S)-2-fluorhexanoat;

Beispiel 3

0,2 g (S)-2-Fluorhexansäure (S-α-Fluorcapronsäure), 0,8 g trans-4-(2-[4-(4-Dodecyloxybenzoyloxy)phenyl]-äthyl)cyclohexanol, 0,45 g N,N′-Dicyclohexylcarbodiimid, 3 mg 4-(Dimethylamino)pyridin und 30 ml Dichlormethan wurden in analoger Weise zu Beispiel 1 umgesetzt. Dies ergab 0,6 g trans-4-(2-[4-(4-Dodecyloxybenzoyloxy)phenyl]äthyl)cyclohexyl-(S)-2-fluorhexanoat.

Das als Ausgangsmaterial verwendete trans-4-(2-[4-(4-Dodecyloxybenzoyloxy)phenyl]äthyl)cyclohexanol wurde wie folgt hergesellt:

(a) Ein Gemisch aus 5 g trans-4-Hydroxycyclohexancarboxaldehyd, 30 g 4-Methoxybenzyl-triphenylphosphoniumbromid, und 250 ml tert. Butyl-methyläther wurde unter Argonbegasung bei 0 °C mit 7 g Kalium-tert. butylat versetzt. Das Reaktionsgemisch wurde noch 2 Stunden bei Raumtemperatur gerührt und dann mit Wasser versetzt. Das Reaktionsgemisch wurde dreimal mit je 50 ml Diäthyläther extrahiert. Die vereinigten organischen Phasen wurden mit Wasser gewaschen, über Magnesiumsulfat getrocknet, filtriert und anschliessend eingeengt. Der Rückstand wurde mehrmals mit Hexan behandelt. Die vereinigten organischen Phasen wurden nochmals eingeengt. Das erhaltene Rohprodukt wurde an Kieselgel mit Hexan chromatographisch gereinigt. Dies ergab 8 g 1-(trans-4-Hydroxycyclohexyl)-2-(4-methoxyphenyl)-äthen (cis/trans-Gemisch).

(b) Ein Gemisch von 8 g 1-(trans-4-Hydroxycyclohexyl)-2-(4-methoxyphenyl)äthen, 0,5 g Palladium/Kohle (5%) und 100 ml Aethylacetat wurde unter Einleitung von Wasserstoff in analoger Weise zu Beispiel 1 (d) umgesetzt. Dies ergab 7,5 g trans-4-(2-[4-Methoxyphenyl]äthyl)cyclohexanol.

(c) Ein Gemisch von 7,5 g trans-4-(2-[4-Methoxyphenyl]äthyl)cyclohexanolund 100 ml Dichlormethan wurde bei 0 °C mit 27 ml einer 1 M Lösung von Bortribromid in Dichlormethan versetzt. Das Reaktionsgemisch wurde noch 2 Stunden bei Raumtemperatur gerührt und dann auf Eiswasser gegossen. Die organische Phase wurde abgetrennt und die wässrige Phase dreimal mit je 50 ml Dichlormethan nachextrahiert. Die vereinigten organischen Phasen werden mit Wasser, Natriumhydrogencarbonat-Lösung und nochmals mit Wasser gewaschen und dan über Magnesiumsulfat getrocknet, filtriert und eingeengt. Der Rückstand wurde aus Hexan umkristallisiert und ergab 5,2 g trans-4-(2-[4-Hydroxyphenyl]äthyl)cyclohexanol.

(d) 0,7 g 4-Dodecyloxybenzoesäure, 0,5 g trans-4-(2-[4-Hydroxyphenyl]äthyl)cyclohexanol, 0,6 g N,N′-Dicyclohexylcarbodiimid, 4 mg 4-(Dimethylamino)pyridin und 50 ml Dichlormethan wurden in analoger Weise zu Beispiel 1 umgesetzt. Dies ergab 0,8 g trans-4-(2-[4-(4-Dodecyloxybenzoyloxy)phenyl]äthyl)-cyclohexanol.

In analoger Weise können die folgenden Verbindungen hergestellt werden:

trans-4-(2-[4-(4-Methoxybenzoyloxy)phenyl]äthyl)cyclohexyl-(S)-2-fluorhexanoat;
trans-4-(2-[4-(4-Aethoxybenzoyloxy)phenyl]äthyl)cyclohexyl-(S)-2-fluorhexanoat;
trans-4-(2-[4-(4-Propyloxybenzoyloxy)phenyl]äthyl)cyclohexyl-(S)-2-fluorhexanoat;
trans-4-(2-[4-(4-Butyloxybenzoyloxy)phenyl]äthyl)cyclohexyl-(S)-2-fluorhexanoat;
trans-4-(2-[4-(4-Pentyloxybenzoyloxy)phenyl]äthyl)cyclohexyl-(S)-2-fluorhexanoat;
trans-4-(2-[4-Hexyloxybenzoyloxy)phenyl]äthyl)cyclohexyl-(S)-2-fluorhexanoat;
trans-4-(2-[4-(4-Heptyloxybenzoyloxy)phenyl]äthyl)cyclohexyl-(S)-2-fluorhexanoat;
trans-4-(2-[4-(4-Octyloxybenzoyloxy)phenyl]äthyl)cyclohexyl-(S)-2-fluorhexanoat;

trans-4-(2-[4-(4-Nonyloxybenzoyloxy)phenyl]äthyl)cyclohexyl-(S)-2-fluorhexanoat;
trans-4-(2-[4-(4-Decyloxybenzoyloxy)phenyl]äthyl)cyclohexyl-(S)-2-fluorhexanoat;
trans-4-(2-[4-(4-Undecyloxybenzoyloxy)phenyl]äthyl)cyclohexyl-(S)-2-fluorhexanoat;
trans-4-(2-[4-(4-Methylbenzoyloxy)phenyl]äthyl-cyclohexyl-(S)-2-fluorhexanoat;
trans-4-(2-[4-(4-Aethylbenzoyloxy)phenyl]äthyl-cyclohexyl-(S)-2-fluorhexanoat;
trans-4-(2-[4-(4-Propylbenzoyloxy)phenyl]äthyl-cyclohexyl-(S)-2-fluorhexanoat;
trans-4-(2-[4-(4-Butylbenzoyloxy)phenyl]äthyl-cyclohexyl-(S)-2-fluorhexanoat;
trans-4-(2-[4-(4-Pentylbenzoyloxy)phenyl]äthyl-cyclohexyl-(S)-2-fluorhexanoat;
trans-4-(2-[4-(4-Hexylbenzoyloxy)phenyl]äthyl-cyclohexyl-(S)-2-fluorhexanoat;
trans-4-(2-[4-(4-Heptylbenzoyloxy)phenyl]äthyl-cyclohexyl-(S)-2-fluorhexanoat;
trans-4-(2-[4-(4-Octylbenzoyloxy)phenyl]äthyl-cyclohexyl-(S)-2-fluorhexanoat;
trans-4-(2-[4-(4-Nonylbenzoyloxy)phenyl]äthyl-cyclohexyl-(S)-2-fluorhexanoat;
trans-4-(2-[4-(4-Decylbenzoyloxy)phenyl]äthyl-cyclohexyl-(S)-2-fluorhexanoat;
trans-4-(2-[4-(2-Fluor-4-methoxybenzoyloxy)phenyl]äthyl)cyclohexyl-(S)-2-fluorhexanoat;
trans-4-(2-[4-(2-Fluor-4-äthoxybenzoyloxy)phenyl]äthyl)cyclohexyl-(S)-2-fluorhexanoat;
trans-4-(2-[4-(2-Fluor-4-propyloxybenzoyloxy)phenyl]äthyl)cyclohexyl-(S)-2-fluorhexanoat;
trans-4-(2-[4-(2-Fluor-4-butyloxybenzoyloxy)phenyl]äthyl)cyclohexyl-(S)-2-fluorhexanoat;
trans-4-(2-[4-(2-Fluor-4-pentyloxybenzoyloxy)phenyl]äthyl)cyclohexyl-(S)-2-fluorhexanoat;
trans-4-(2-[4-(2-Fluor-4-hexyloxybenzoyloxy)phenyl]äthyl)cyclohexyl-(S)-2-fluorhexanoat;
trans-4-(2-[4-(2-Fluor-4-heptyloxybenzoyloxy)phenyl]äthyl)cyclohexyl-(S)-2-fluorhexanoat;
trans-4-(2-[4-(2-Fluor-4-octyloxybenzoyloxy)phenyl]äthyl)cyclohexyl-(S)-2-fluorhexanoat;
trans-4-(2-[4-(2-Fluor-4-nonyloxybenzoyloxy)phenyl]äthyl)cyclohexyl-(S)-2-fluorhexanoat;
trans-4-(2-[4-(2-Fluor-4-decyloxybenzoyloxy)phenyl]äthyl)cyclohexyl-(S)-2-fluorhexanoat;
trans-4-(2-[4-(2-Fluor-4-undecyloxybenzoyloxy)phenyl]äthyl)cyclohexyl-(S)-2-fluorhexanoat;
trans-4-(2-[4-(2-Fluor-4-dodecyloxybenzoyloxy)phenyl]äthyl)cyclohexyl-(S)-2-fluorhexanoat;
trans-4-(2-[4-(3-Fluor-4-methoxybenzoyloxy)phenyl]äthyl)cyclohexyl-(S)-2-fluorhexanoat;
trans-4-(2-[4-(3-Fluor-4-äthoxybenzoyloxy)phenyl]äthyl)cyclohexyl-(S)-2-fluorhexanoat;
trans-4-(2-[4-(3-Fluor-4-propyloxybenzoyloxy)phenyl]äthyl)cyclohexyl-(S)-2-fluorhexanoat;
trans-4-(2-[4-(3-Fluor-4-butyloxybenzoyloxy)phenyl]äthyl)cyclohexyl-(S)-2-fluorhexanoat;
trans-4-(2-[4-(3-Fluor-4-pentyloxybenzoyloxy)phenyl]äthyl)cyclohexyl-(S)-2-fluorhexanoat;
trans-4-(2-[4-(3-Fluor-4-hexyloxybenzoyloxy)phenyl]äthyl)cyclohexyl-(S)-2-fluorhexanoat;
trans-4-(2-[4-(3-Fluor-4-heptyloxybenzoyloxy)phenyl]äthyl)cyclohexyl-(S)-2-fluorhexanoat;
trans-4-(2-[4-(3-Fluor-4-octyloxybenzoyloxy)phenyl]äthyl)cyclohexyl-(S)-2-fluorhexanoat;
trans-4-(2-[4-(3-Fluor-4-nonyloxybenzoyloxy)phenyl]äthyl)cyclohexyl-(S)-2-fluorhexanoat;
trans-4-(2-[4-(3-Fluor-4-decyloxybenzoyloxy)phenyl]äthyl)cyclohexyl-(S)-2-fluorhexanoat;
trans-4-(2-[4-(3-Fluor-4-undecyloxybenzoyloxy)phenyl]äthyl)cyclohexyl-(S)-2-fluorhexanoat;
trans-4-(2-[4-(3-Fluor-4-dodecyloxybenzoyloxy)phenyl]äthyl)cyclohexyl-(S)-2-fluorhexanoat;
trans-4-(2-[4-(2-Chlor-4-dodecyloxybenzoyloxy)phenyl]äthyl)cyclohexyl-(S)-2-fluorhexanoat;
trans-4-(2-[4-(3-Chlor-4-dodecyloxybenzoyloxy)phenyl]äthyl)cyclohexyl-(S)-2-fluorhexanoat;
trans-4-(2-[4-(2-Brom-4-dodecyloxybenzoyloxy)phenyl]äthyl)cyclohexyl-(S)-2-fluorhexanoat;
trans-4-(2-[4-(3-Brom-4-dodecyloxybenzoyloxy)phenyl]äthyl)cyclohexyl-(S)-2-fluorhexanoat;
trans-4-(2-[4-(2-Cyano-4-dodecyloxybenzoyloxy)phenyl]äthyl)cyclohexyl-(S)-2-fluorhexanoat;
trans-4-(2-[4-(3-Cyano-4-dodecyloxybenzoyloxy)phenyl]äthyl)cyclohexyl-(S)-2-fluorhexanoat;
trans-4-(2-[4-(2-Chlor-4-dodecyloxybenzoyloxy)phenyl]äthyl)cyclohexyl-(R)-2-fluorhexanoat;
trans-4-(2-[4-(3-Chlor-4-dodecyloxybenzoyloxy)phenyl]äthyl)cyclohexyl-(R)-2-fluorhexanoat;
trans-4-(2-[4-(2-Brom-4-dodecyloxybenzoyloxy)phenyl]äthyl)cyclohexyl-(R)-2-fluorhexanoat;
trans-4-(2-[4-(3-Brom-4-dodecyloxybenzoyloxy)phenyl]äthyl)cyclohexyl-(R)-2-fluorhexanoat;
trans-4-(2-[4-(2-Cyano-4-dodecyloxybenzoyloxy)phenyl]äthyl)cyclohexyl-(R)-2-fluorhexanoat;
trans-4-(2-[4-(3-Cyano-4-dodecyloxybenzoyloxy)phenyl]äthyl)cyclohexyl-(R)-2-fluorhexanoat;
trans-4-(2-[4-(4-[(S)-2-Chlor-1-propyloxy]benzoyloxy)phenyl]äthyl)cyclohexyl-(S)-2-fluorhexanoat;
trans-4-(2-[4-(4-[(R)-2-Chlor-1-propyloxy]benzoyloxy)phenyl]äthyl)cyclohexyl-(S)-2-fluorhexanoat;
trans-4-(2-[4-(4-[(S)-2-Chlor-1-propyloxy]benzoyloxy)phenyl]äthyl)cyclohexyl-(R)-2-fluorhexanoat;
trans-4-(2-[4-(4-[(R)-2-Chlor-1-propyloxy]benzoyloxy)phenyl[äthyl)cyclohexyl-(R)-2-fluorhexanoat;
trans-4-(4-[4-Methoxybenzoyloxy]phenyl)cyclohexyl-(S)-2-fluorhexanoat;
trans-4-(4-[4-Aethoxybenzoyloxy]phenyl)cyclohexyl-(S)-2-fluorhexanoat;
trans-4-(4-[4-Propyloxybenzoyloxy]phenyl)cyclohexyl-(S)-2-fluorhexanoat;
trans-4-(4-[4-Butyloxybenzoyloxy]phenyl)cyclohexyl-(S)-2-fluorhexanoat;
trans-4-(4-[4-Pentyloxybenzoyloxy]phenyl)cyclohexyl-(S)-2-fluorhexanoat;

trans-4-(4-[4-Hexyloxybenzoyloxy]phenyl)cyclohexyl-(S)-2-fluorhexanoat;
trans-4-(4-[4-Heptyloxybenzoyloxy]phenyl)cyclohexyl-(S)-2-fluorhexanoat;
trans-4-(4-[4-Octyloxybenzoyloxy]phenyl)cyclohexyl-(S)-2-fluorhexanoat;
trans-4-(4-[4-Nonyloxybenzoyloxy]phenyl)cyclohexyl-(S)-2-fluorhexanoat;
trans-4-(4-[4-Decyloxybenzoyloxy]phenyl)cyclohexyl-(S)-2-fluorhexanoat;
trans-4-(4-[4-Undecyloxybenzoyloxy]phenyl)cyclohexyl-(S)-2-fluorhexanoat;
trans-4-(4-[4-Dodecyloxybenzoyloxy]phenyl)cyclohexyl-(S)-2-fluorhexanoat, Smp. (C-$S_B$) 64 ° C, Umwandlung $S_B$-$S_A$ 113 ° C, Klp. ($S_A$-I) 159 ° C;
trans-4-(4-[3-Fluor-4-dodecyloxybenzoyloxy]phenyl)cyclohexyl-(S)-2-fluorhexanoat, Smp. (C-$S_C$*) 73 ° C, Klp. ($S_C$*-I) 148 ° ;
trans-4-(4-[3-Chlor-4-dodecyloxybenzoyloxy]phenyl)cyclohexyl-(S)-2-fluorhexanoat, Smp. (C-$S_C$*) 50 ° C, Klp. ($S_C$*-I) 138 ° C;
trans-4-(4-[3-Brom-4-dodecyloxybenzoyloxy]phenyl)cyclohexyl-(S)-2-fluorhexanoat, Smp. (C-$S_C$*)56 %, Klp. ($S_C$*-I)118 ° C;
trans-4-(4-[3-Cyano-4-dodecyloxybenzoyloxy]phenyl)cyclohexyl-(S)-2-fluorhexanoat;
trans-4-(4-[2,3-Difluor-4-methoxybenzoyloxy]phenyl)cyclohexyl-(S)-2-fluorhexanoat;
trans-4-(4-[2,3-Difluor-4-äthoxybenzoyloxy]phenyl)cyclohexyl-(S)-2-fluorhexanoat;
trans-4-(4-[2,3-Difluor-4-propyloxybenzoyloxy]phenyl)cyclohexyl-(S)-2-fluorhexanoat;
trans-4-(4-[2,3-Difluor-4-butyloxybenzoyloxy]phenyl)cyclohexyl-(S)-2-fluorhexanoat;
trans-4-(4-[2,3-Difluor-4-pentyloxybenzoyloxy]phenyl)cyclohexyl-(S)-2-fluorhexanoat;
trans-4-(4-[2,3-Difluor-4-hexyloxybenzoyloxy]phenyl)cyclohexyl-(S)-2-fluorhexanoat;
trans-4-(4-[2,3-Difluor-4-heptyloxybenzoyloxy]phenyl)cyclohexyl-(R)-2-fluorfhexanoat, Smp. (C-$S_C$*) 82 ° C, Umwandlung ($S_C$*$S_A$)93 ° C, Umwandlung ($S_A$-Ch) 140 ° C, Klp. (Ch-I) 151 ° C;
trans-4-(4-[2,3-Difluor-4-octyloxybenzoyloxy]phenyl)cyclohexyl-(S)-2-fluorhexanoat, Smp. (C-$S_C$*)77 ° C, Umwandlung ($S_C$*-$S_A$) 91 ° C, Umwandlung ($S_A$-Ch)143 ° C. Klp. (Ch-I) 150 ° C;
trans-4-(4-[2,3-Difluor-4-nonyloxybenzoyloxy]phenyl)cyclohexyl-(R)-2-fluorhexanoat, Smp. (C-$S_C$*) 74 ° C, Umwandlung ($S_C$*-$S_A$) 87 ° C, Umwandlung ($S_A$-Ch) 145 ° C, Klp, (Ch-I) 148 ° C;
trans-4-(4-[2,3-Difluor-4-decyloxybenzoyloxy]phenyl)cyclohexyl-(S)-2-fluorhexanoat, Smp. (C-$S_C$*) 72 ° C, Umwandlung ($S_C$*-$S_A$) 84 ° C, Umwandlung ($S_A$-Ch) 147 ° C, Klp (Ch-I) 148 ° C;
trans-4-(4-[2,3-Difluor-4-undecyloxybenzoyloxy]phenyl)cyclohexyl-(S)-2-fluorhexanoat, Smp. (C-$S_A$) 78 ° C, Umwandlung ($S_C$*-$S_A$) 77 ° C, Klp. ($S_A$-I) 147 ° C;
trans-4-(4-[2,3-Difluor-4-dodecyloxy]benzoyloxy]phenyl)cyclohexyl-(S)-2-fluorhexanoat, Smp. (C-$S_C$*) 73 ° C, Umwandlung ($S_C$*-$S_A$) 74 ° C, Klp. ($S_A$-I) 147 ° C;
trans-4-(4-[2,3-Difluor-4-methoxybenzoyloxy]phenyl)cyclohexyl-(2S,3S)-2-fluor-3-methylpentanoat;
trans-4-(4-[2,3-Difluor-4-äthoxybenzoyloxy]phenyl)cyclohexyl-(2S,3S)-2-fluor-3-methylpentanoat;
trans-4-(4-[2,3-Difluor-4-propyloxybenzoyloxy]phenyl)cyclohexyl-(2S,3S)-2-fluor-3-methylpentanoat;
trans-4-(4-[2,3-Difluor-4-butyloxybenzoyloxy]phenyl)cyclohexyl-(2S,3S)-2-fluor-3-methylpentanoat;
trans-4-(4-[2,3-Difluor-4-pentyloxybenzoyloxy]phenyl)cyclohexyl-(2S,3S)-2-fluor-3-methylpentanoat;
trans-4-(4-[2,3-Difluor-4-hexyloxybenzoyloxy]phenyl)cyclohexyl-(2S,3S)-2-fluor-3-methylpentanoat;
trans-4-(4-[2,3-Difluor-4-heptyloxybenzoyloxy]phenyl)cyclohexyl-(2S,3S)-2-fluor-3-methylpentanoat;
trans-4-(4-[2,3-Difluor-4-octyloxybenzoyloxy]phenyl)cyclohexyl-(2S,3S)-2-fluor-3-methylpentanoat;
trans-4-(4-[2,3-Difluor-4-nonyloxybenzoyloxy]phenyl)cyclohexyl-(2S,3S)-2-fluor-3-methylpentanoat;
trans-4-(4-[2,3-Difluor-4-decyloxybenzoyloxy]phenyl)cyclohexyl-(2S,3S)-2-fluor-3-methylpentanoat;
trans-4-(4-[2,3-Difluor-4-undecyloxybenzoyloxy]phenyl)cyclohexyl-(2S,3S)-2-fluor-3-methylpentanoat;
trans-4-(4-[2,3-Difluor-4-dodecyloxybenzoyloxy]phenyl)cyclohexyl-(2S,3S)-2-fluor-3-methylpentanoat;
trans-4-[(4-[4-Methoxybenzoyloxy]phenoxy)methyl]cyclohexyl-(S)-2-fluorhexanoat;
trans-4-[(4-[Aethoxybenzoyloxy]phenoxy)methyl]cyclohexyl-(S)-2-fluorhexanoat;
trans-4-[(4-[4-Propyloxybenzoyloxy]phenoxy)methyl]cyclohexyl-(S)-2-fluorhexanoat;
trans-4-[(4-[4-Butyloxybenzoyloxy]phenoxy)methyl]cyclohexyl-(S)-2-fluorhexanoat;
trans-4-[(4-[4-Pentyloxybenzoyloxy]phenoxy)methyl]cyclohexyl-(S)-2-fluorhexanoat;
trans-4-[(4-[4-Hexyloxybenzoyloxy]phenoxy)methyl]cyclohexyl-(S)-2-fluorhexanoat;
trans-4-[(4-[4-Heptyloxybenzoyloxy]phenoxy)methyl]cyclohexyl-(S)-2-fluorhexanoat;
trans-4-[(4-[4-Octyloxybenzoyloxy]phenoxy)methyl]cyclohexyl-(S)-2-fluorhexanoat;
trans-4-[(4-[4-Nonyloxybenzoyloxy]phenoxy)methyl]cyclohexyl-(S)-2-fluorhexanoat;
trans-4-[(4-[4-Decyloxybenzoyloxy]phenoxy)methyl]cyclohexyl-(S)-2-fluorhexanoat;
trans-4-[(4-[4-Undecyloxybenzoyloxy]phenoxy)methyl]cyclohexyl-(S)-2-fluorhexanoat;
trans-4-[(4-[4-Dodecyloxybenzoyloxy]phenoxy)methyl]cyclohexyl-(S)-2-fluorhexanoat;
trans-4-[(4-[3-Fluor-4-dodecyloxybenzoyloxy]phenoxy)methyl]cyclohexyl-(S)-2-fluorhexanoat;

trans-4-[(4-[3-Chlor-4-dodecyloxybenzoyloxy]phenoxy)methyl]cyclohexyl-(S)-2-fluorhexanoat;
trans-4-[(4-[3-Brom-4-dodecyloxybenzoyloxy]phenoxy)methyl]cyclohexyl-(S)-2-fluorhexanoat;
trans-4-[(4-[3-Cyano-4-dodecyloxybenzoyloxy]phenoxy)methyl]cyclohexyl-(S)-2-fluorhexanoat;
trans-4-[(4-[2,3-Difluor-4-methoxybenzoyloxy]phenoxy)methyl]cyclohexyl-(S)-2-fluorhexanoat;
trans-4-[(4-[2,3-Difluor-4-äthoxybenzoyloxy]phenoxy)methyl]cyclohexyl-(S)-2-fluorhexanoat;
trans-4-[(4-[2,3-Difluor-4-propyloxybenzoyloxy]phenoxy)methyl]cyclohexyl-(S)-2-fluorhexanoat;
trans-4-[(4-[2,3-Difluor-4-butyloxybenzoyloxy]phenoxy)methyl]cyclohexyl-(S)-2-fluorhexanoat:
trans-4-[(4-[2,3-Difluor-4-pentyloxybenzoyloxy]phenoxy)methyl)cyclohexyl-(S)-2-fluorhexanoat:
trans-4-[(4-[2,3-Difluor-4-hexyloxybenzoyloxy]phenoxy)methyl]cyclohexyl-(S)-2-fluorhexanoat:
trans-4-[(4-[2,3-Difluor-4-heptyloxybenzoyloxy]phenoxy)methyl]cyclohexyl-(S)-2-fluorhexanoat;
trans-4-[(4-[2,3-Difluor-4-octyloxybenzoyloxy]phenoxy)methyl]cyclohexyl-(S)-2-fluorhexanoat;
trans-4-[(4-[2,3-Difluor-4-nonyloxybenzoyloxy]phenoxy)methyl]cyclohexyl-(S)-2-fluorhexanoat;
trans-4-[(4-[2,3-Difluor-4-decyloxybenzoyloxy]phenoxy)methyl]cyclohexyl-(S)-2-fluorhexanoat:
trans-4-[(4-[2,3-Difluor-4-undecyloxybenzoyloxy]phenoxy)methyl]cyclohexyl-(S)-2-fluorhexanoat;
trans-4-[(4-[2,3-Difluor-4-dodecyloxybenzoyloxy]phenoxy)methyl]cyclohexyl-(S)2-fluorhexanoat;

Beispiel 4

0,23 g (S)-2-Fluorhexansäure (S-α-Fluorcapronsäure), 0,24 g trans-4-(4-[(trans -4-Hydroxycyclohexyl)-methoxy]phenyl)cyclohexanol, 0,29 g N,N′-Dicyclohexylcarbodiimid, 0,02 g 4-(Dimethylamino)pyridin und 20 ml Dichlormethan wurden in analoger Weise zu Beispiel 1 umgesetzt. Dies ergab 0,2 g trans-4-(4-[(trans-4-[(S) -2-Fluorhexanoyloxy]cyclohexyl)methoxy]phenyl)cyclohexyl -(S)-2-fluorhexanoat; Smp. (C-S) <25 °C, Umwandlung (S-$S_A$) 67 °C, Klp. (S-$S_A$-I) 88.9 °C.

Das als Ausgangsmaterial verwendete trans-4-(4-[(trans -4-Hydroxycyclohexyl)methoxy]phenyl)cyclohexanol wurde wie folgt hergestellt:

(a) Ein Gemisch aus 1,0 g 4-(4-Hydroxyphenyl)cyclohexanon, 1,4 g (trans-4-Hydroxycyclohexyl)methyl-tosylat, 1,5 g Kaliumcarbonat und 60 ml absolutem Butanon wurde 2 Tage unter Rückfluss erwärmt. Anschliessend wurde das abgekülte Reaktionsgemisch auf Wasser gegossen und dreimal mit je 50 ml Dichlormethan extrahiert. Die vereinigten organischen Phasen wurden mit Wasser gewaschen, über Magnesiumsulfat getrocknet, filtriert und anschliessend eingeengt. Der Rückstand wurde an Kieselgel mit Toluol/Aethylacetat (Vol. 5:1) chromatographisch gereinigt. Dies ergab 1,0 g 4-(4-[(trans-4-Hydroxycyclohexyl)methoxy]phenyl)cyclohexanon.

(b). 1,0 g 4-(4-[(trans-4-Hydroxycyclohexyl)methoxy]phenyl)cyclohexanon, 0,12 g Natriumborhydrid, 20 ml Methanol und 2 ml Diäthyläther wurden in analoger Weise zu Beispiel 1 (a) umgesetzt. Das Rohprodukt wurde an Kieselgel mit Dichlormethan/Methanol (Vol 19:1) chromatographische gereinigt. Dies ergab 0,24 g trans-4-(4-[(trans -4-Hydroxycyclohexyl)methoxy]phenyl)cyclohexanol.

In analoger Weise können die folgenden Verbindungen hergestellt werden:

trans-4-(4-[(trans-4-[(S)-2-fluorpropanoyloxy]cyclohexyl)methoxy]phenyl)cyclohexyl-(S)-2-fluorpropanoat;
trans-4-(4-[(trans-4-[(S)-2-fluorbutanoyloxy]cyclohexyl)methoxy]phenyl)cyclohexyl-(S)-2-fluorbutanoat;
trans-4-(4-[(trans-4-[(S)-2-fluorpentanoyloxy]cyclohexyl)methoxy]phenyl)cyclohexyl-(S)-2-fluorpentanoat;
trans-4-(4-[(trans-4-[(S)-2-fluorheptanoyloxy]cyclohexyl)methoxy]phenyl)cyclohexyl-(S)-2-fluorheptanoat;
trans-4-(4-[(trans-4-[(S)-2-fluoroctanoyloxy]cyclohexyl)methoxy]phenyl)cyclohexyl-(S)-2-fluoroctanoat;
trans-4-(4-[(trans-4-[(S)-2-fluornonanoyloxy]cyclohexyl)methoxy]phenyl)cyclohexyl-(S)-2-fluornonanoat;
trans-4-(4-[(trans-4-[(S)-2-fluordecanoyloxy]cyclohexyl)methoxy]phenyl)cyclohexyl-(S)-2-fluordecanoat;
trans-4-(4-[(trans-4-[(R)-2-fluorpropanoyloxy]cyclohexyl)methoxy]phenyl)cyclohexyl-(R)-2-fluorpropanoat;
trans-4-(4-[(trans-4-[(R)-2-fluorbutanoyloxy]cyclohexyl)methoxy]phenyl)cyclohexyl-(R)-2-fluorbutanoat;
trans-4-(4-[(trans-4-[(R)-2-fluorpentanoyloxy]-cyclohexyl)methoxy]phenyl)cyclohexyl-(R)-2-fluorpentanoat;
trans-4-(4-[(trans-4-[(R)-2-fluorhexanoyloxy]cyclohexyl)methoxy]phenyl)cyclohexyl-(R)-2-fluorhexanoat;
trans-4-(4-[(trans-4-[(R)-2-fluorheptanoyloxy]cyclohexyl)methoxy]phenyl)cyclohexyl-(R)-2-fluorheptanoat;
trans-4-(4-[(trans-4-[(R)-2-fluoroctanoyloxy]cyclohexyl)methoxy]phenyl)cyclohexyl-(R)-2-fluoroctanoat;
trans-4-(4-[(trans-4-[(R)-2-fluornonanoyloxy]cyclohexyl)methoxy]phenyl)cyclohexyl-(R)-2-fluornonanoat;
trans-4-(4-[(trans-4-[(R)-2-fluordecanoyloxy]cyclohexyl)methoxy]phenyl)cyclohexyl-(R)-2-fluordecanoat;
trans-4-(2-[4-(trans-4-[(S)-2-fluorpropanoyloxy]cyclohexyl)phenyl]äthyl)cyclohexyl-(S)-2-fluorpropanoat;
trans-4-(2-[4-(trans-4-[(S)-2-fluorbutanoyloxy]cyclohexyl)phenyl]äthyl)cyclohexyl-(S)-2-fluorbutanoat;
trans-4-(2-[4-(trans-4-[(S)-2-fluorpentanoyloxy]cyclohexyl)phenyl]äthyl)cyclohexyl-(S)-2-fluorpentanoat;
trans-4-(2-[4-(trans-4-[(S)-2-fluorhexanoyloxy]cyclohexyl)phenyl]äthyl)cyclohexyl-(S)-2-fluorhexanoat;
trans-4-(2-[4-(trans-4-[(S)-2-fluorheptanoyloxy]cyclohexyl)phenyl]äthyl)cyclohexyl-(S)-2-fluorheptanoat;
trans-4-(2-[4-(trans-4-[(S)-2-fluoroctanoyloxy]cyclohexyl)phenyl]äthyl)cyclohexyl-(S)-2-fluoroctanoat;

trans-4-(2-[4-(trans-4-[(S)-2-fluornonanoyloxy]cyclohexyl)phenyl]äthyl)cyclohexyl-(S)-2-fluornonanoat;
trans-4-(2-[4-(trans-4-[(S)-2-fluordecanoyloxy]cyclohexyl)phenyl]äthyl)cyclohexyl-(S)-2-fluordecanoat;
trans-4, trans-4′-Di[(S)-2-fluorpropanoyloxy]bicyclohexyl;
trans-4, trans-4′-Di[(S)-2-fluorbutanoyloxy]bicyclohexyl;
trans-4, trans-4′-Di[(S)-2-fluorpentanoyloxy]bicyclohexyl;
trans-4, trans-4′-Di[(S)-2-fluorhexanoyloxy]bicyclohexyl; Smp. (C-I) 38,1 °C, Umwandlung (S-S), 18,8 °C, Klp. (S-I) 22,3 °C;
trans-4, trans-4′-Di[(S)-2-fluorheptanoyloxy]-bicyclohexyl;
trans-4, trans-4′-Di[(S)-2-fluoroctanoyloxy]bicyclohexyl;
trans-4, trans-4′-Di[(S)-2-fluornonanoyloxy]bicyclohexyl;
trans-4, trans-4′-Di[(S)-2-fluordecanoyloxy]bicyclohexyl;
trans-4, trans-4′-Di[(R)-2-fluorpropanoyloxy]bicyclohexyl;
trans-4, trans-4′-Di[(R)-2-fluorbutanoyloxy]bicyclohexyl;
trans-4, trans-4′-Di[(R)-2-fluorpentanoyloxy]bicyclohexyl;
trans-4, trans-4′-Di[(R)-2-fluorhexanoyloxy]bicyclohexyl;
trans-4, trans-4′-Di[(R)-2-fluorheptanoyloxy]bicyclohexyl;
trans-4, trans-4′-Di[(R)-2-fluoroctanoyloxy]bicyclohexyl;
trans-4, trans-4′-Di[(R)-2-fluornonanoyloxy]bicyclohexyl;
trans-4, trans-4′-Di[(R)-2-fluordecanoyloxy]bicyclohexyl;
1,4-Bis(trans-4-[(S)-2-fluorpropanoyloxy]cyclohexyl)benzol;
1,4-Bis(trans-4-[(S)-2-fluorbutanoyloxy]cyclohexyl)benzol;
1,4-Bis(trans-4-[(S)-2-fluorpentanoyloxy]cyclohexyl)benzol;
1,4-Bis(trans-4-[(S)-2-fluorhexanoyloxy]cyclohexyl)benzol;
1,4-Bis(trans-4-[(S)-2-fluorheptanoyloxy]cyclohexyl)benzol;
1,4-Bis(trans-4-[(S)-2-fluoroctanoyloxy]cyclohexyl)benzol;
1,4-Bis(trans-4-[(S)-2-fluornonanoyloxy]cyclohexyl)benzol;
1,4-Bis(trans-4-[(S)-2-fluordecanoyloxy]cyclohexyl)benzol;
2-Fluor-1,4-bis(trans-4-[(S)-2-fluorpropanoyloxy]cyclohexyl)benzol;
2-Fluor-1,4-bis(trans-4-[(S)-2-fluorbutanoyloxy]cyclohexyl)benzol;
2-Fluor-1,4-bis(trans-4-[(S)-2-fluorpentanoyloxy]cyclohexyl)benzol;
2-Fluor-1,4-bis(trans-4-[(S)-2-fluorhexanoyloxy]cyclohexyl)benzol;
2-Fluor-1,4-bis(trans-4-[(S)-2-fluorheptanoyloxy]cyclohexyl)benzol;
2-Fluor-1,4-bis(trans-4-[(S)-2-fluoroctanoyloxy]cyclohexyl)benzol;
2-Fluor-1,4-bis(trans-4-[(S)-2-fluornonanoyloxy]cyclohexyl)benzol;
2-Fluor-1,4-bis(trans-4-[(S)-2-fluordecanoyloxy]cyclohexyl)benzol;
2-Chlor-1,4-bis(trans-4-[(S)-2-fluorpropanoyloxy]cyclohexyl)benzol;
2-Chlor-1,4-bis(trans-4-[(S)-2-fluorbutanoyloxy]cyclohexyl)benzol;
2-Chlor-1,4-bis(trans-4-[(S)-2-fluorpentanoyloxy]cyclohexyl)benzol;
2-Chlor-1,4-bis(trans-4-[(S)-2-fluorhexanoyloxy]cyclohexyl)benzol;
2-Chlor-1,4-bis(trans-4-[(S)-2-fluorheptanoyloxy]cyclohexyl)benzol;
2-Chlor-1,4-bis(trans-4-[(S)-2-fluoroctanoyloxy]cyclohexyl)benzol;
2-Chlor-1,4-bis(trans-4-[(S)-2-fluornonanoyloxy]cyclohexyl)benzol;
2-Chlor-1,4-bis(trans-4-[(S)-2-fluordecanoyloxy]cyclohexyl)benzol;
2-Brom-1,4-bis(trans-4-[(S)-2-fluorpropanoyloxy]cyclohexyl)benzol;
2-Brom-1,4-bis(trans-4-[(S)-2-fluorbutanoyloxy]cyclohexyl)benzol;
2-Brom-1,4-bis(trans-4-[(S)-2-fluorpentanoyloxy]cyclohexyl)benzol;
2-Brom-1,4-bis(trans-4-[(S)-2-fluorhexanoyloxy]cyclohexyl)benzol;
2-Brom-1,4-bis(trans-4-[(S)-2-fluorheptanoyloxy]cyclohexyl)benzol;
2-Brom-1,4-bis(trans-4[(S)-2-fluoroctanoyloxy]cyclohexyl)benzol;
2-Brom-1,4-bis(trans-4-[(S)-2-fluornonanoyloxy]cyclohexyl)benzol;
2-Brom-1,4-bis(trans-4-[(S)-2-fluordecanoyloxy]cyclohexyl)benzol;
2-Cyano-1,4-bis(trans-4-[(S)-2-fluorpropanoyloxy]cyclohexyl)benzol;
2-Cyano-1,4-bis(trans-4-[(S)-2-fluorbutanoyloxy]cyclohexyl)benzol;
2-Cyano-1,4-bis(trans-4-[(S)-2-fluorpentanoyloxy]cyclohexyl)benzol;
2-Cyano-1,4-bis(trans-4-[(S)-2-fluorhexanoyloxy]cyclohexyl)benzol;
2-Cyano-1,4-bis(trans-4-[(S)-2-fluorheptanoyloxy]cyclohexyl)benzol;
2-Cyano-1,4-bis(trans-4-[(S)-2-fluoroctanoyloxy]cyclohexyl)benzol;
2-Cyano-1,4-bis(trans-4-[(S)-2-fluornonanoyloxy]cyclohexyl)benzol;

2-Cyano-1,4-bis(trans-4-[(S)-2-fluordecanoyloxy]cyclohexyl)benzol;
1,4-Bis(trans-4-[(R)-2-fluorpropanoyloxy]cyclohexyl)benzol;
1,4-Bis(trans-4-[(R)-2-fluorbutanoyloxy]cyclohexyl)benzol;
1,4-Bis(trans-4-[(R)-2-fluorpentanoyloxy]cyclohexyl)benzol;
1,4-Bis(trans-4-[(R)-2-fluorhexanoyloxy]cyclohexyl)benzol;
1,4-Bis(trans-4-[(R)-2-fluorheptanoyloxy]cyclohexyl)benzol;
1,4-Bis(trans-4-[(R)-2-fluoroctanoyloxy]cyclohexyl)benzol;
1,4-Bis(trans-4-[(R)-2-fluornonanoyloxy]cyclohexyl)benzol;
1,4-Bis[(trans-4-[(R)-2-fluordecanoyloxy]cyclohexyl)benzol;
1,4-Bis[(trans-4-[(S)-2-fluorpropanoyloxy]cyclohexyl)methoxy]benzol;
1,4-Bis[(trans-4-[(S)-2-fluorbutanoyloxy]cyclohexyl)methoxy]benzol;
1,4-Bis[(trans-4-[(S)-2-fluorpentanoyloxy]cyclohexyl)methoxy]benzol;
1,4-Bis[(trans-4-[(S)-2-fluorhexanoyloxy]cyclohexyl)methoxy]benzol;
1,4-Bis[(trans-4-[(S)-2-fluorheptanoyloxy]cyclohexyl)methoxy]benzol;
1,4-Bis[(trans-4-[(S)-2-fluoroctanoyloxy]cyclohexyl)methoxy]benzol;
1,4-Bis[(trans-4-[(S)-2-fluornonanoyloxy]cyclohexyl)methoxy]benzol;
1,4-Bis[(trans-4-[(S)-2-fluordecanoyloxy]cyclohexyl)methoxy]benzol;
1,4-Bis[(trans-4-[(R)-2-fluorpropanoyloxy]cyclohexyl)methoxy]benzol;
1,4-Bis[(trans-4-[(R)-2-fluorbutanoyloxy]cyclohexyl)methoxy]benzol;
1,4-Bis[(trans-4-[(R)-2-fluorpentanoyloxy]cyclohexyl)methoxy]benzol;
1,4-Bis[(trans-4-[(R)-2-fluorhexanoyloxy]cyclohexyl)methoxy]benzol;
1,4-Bis[(trans-4-[(R)-2-fluorheptanoyloxy]cyclohexyl)methoxy]benzol;
1,4-Bis[(trans-4-[(R)-2-fluoroctanoyloxy]cyclohexyl)methoxy]benzol;
1,4-Bis[(trans-4-[(R)-2-fluornonanoyloxy]cyclohexyl)methoxy]benzol;
1,4-Bis[(trans-4-[(R)-2-fluordecanoyloxy]cyclohexyl)methoxy]benzol;
2-Fluor-1,4-bis[(trans-4-[(S)-2-fluorhexanoyloxy]cyclohexyl)methoxy]benzol;
2-Chlor-1,4-bis[(trans-4-[(S)-2-fluorhexanoyloxy]cyclohexyl)methoxy]benzol;
2-Brom-1,4-bis[(trans-4-[(S)-2-fluorhexanoyloxy]cyclohexyl)methoxy]benzol;
2-Cyano-1,4-bis[(trans-4-[(S)-2-fluorhexanoyloxy]cyclohexyl)methoxy]benzol;
1,4-Bis[2-(trans-4-[(S)-2-fluorpropanoyloxy]cyclohexyl)äthyl]benzol;
1,4-Bis[2-(trans-4-[(S)-2-fluorbutanoyloxy]cyclohexyl)äthyl]benzol;
1,4-Bis[2-(trans-4-[(S)-2-fluorpentanoyloxy]cyclohexyl)äthyl]benzol;
1,4-Bis[2-(trans-4-[(S)-2-fluorhexanoyloxy]cyclohexyl)äthyl]benzol;
1,4-Bis[2-(trans-4-[(S)-2-fluorheptanoyloxy]cyclohexyl)äthyl]benzol;
1,4-Bis[2-(trans-4-[(S)-2-fluoroctanoyloxy]cyclohexyl)äthyl]benzol;
1,4-Bis[2-(trans-4-[(S)-2-fluornonanoyloxy]cyclohexyl)äthyl]benzol;
1,4-Bis[2-(trans-4-[(S)-2-fluordecanoyloxy]cyclohexyl)äthyl]benzol;
1,4-Bis[2-(trans-4-[(R)-2-fluorpropanoyloxy]cyclohexyl)äthyl]benzol;
1,4-Bis[2-(trans-4-[(R)-2-fluorbutanoyloxy]cyclohexyl)äthyl]benzol;
1,4-Bis[2-(trans-4-[(R)-2-fluorpentanoyloxy]cyclohexyl)äthyl]benzol;
1,4-Bis[2-(trans-4-[(R)-2-fluorhexanoyloxy]cyclohexyl)äthyl]benzol;
1,4-Bis[2-(trans-4-[(R)-2-fluorheptanoyloxy]cyclohexyl)äthyl]benzol;
1,4-Bis[2-(trans-4-[(R)-2-fluoroctanoyloxy]cyclohexyl)äthyl]benzol;
1,4-Bis[2-(trans-4-[(R)-2-fluornonanoyloxy]cyclohexyl)äthyl]benzol;
1,4-Bis[2-(trans-4-[(R)-2-fluordecanoyloxy]cyclohexyl)äthyl]benzol;
trans-4-(4-[(trans-4-[(2S,3S)-2-Fluor-3-methylpentanoyloxy]cyclohexyl)-methoxy]phenyl)cyclohexyl-(2S,3S)-2-fluor-3-methylpentanoat;
trans-4-(2-[4-trans-4-[(2S,3S)-2-Fluor-3-methylpentanoyloxy]cyclohexyl)phenyl]äthyl)cyclohexyl-(2S,3S)-fluor-3-methyl-pentanoat;
trans-4, trans-4′-Di[2S,3S)-2-Fluor-3-methylpentanoyloxy]bicyclohexyl;
1,4,-Bis(trans-4-[(2S,3S)-2-fluor-3-methylpentanoyloxy]biyclohexyl)benzol;
1,4-Bis[(trans-4-[(2S,3S)-2-fluor-3-methylpentanoyloxy]cyclohexyl)methoxy]benzol;
1,4-Bis[2-(trans-4-[(2S,3S)-2-fluor-3-methylpentanoyloxy]cyclohexyl)äthyl]benzol.

Beispiel 5

0,2 g (S)-2-Fluorhexansäure (S-α-Fluorcapronsäure), 0,7 g trans-4-(4′-[(R)-2-Octyl]oxycarbonyl-4-biphenylyl)cyclohexanol, 0,45 g N,N′-Dicyclohexylcarbodiimid, 3 mg 4-(Dimethylamino)pyridin und 30 ml Dichlormethan wurden in analoger Weise zu Beispiel 1 umgesetzt. Dies ergab 0,5 g trans-4-(4′-[(R)-2-Octyl]-oxycarbonyl-4-biphenylyl)cyclohexyl-(S)-2-fluorhexanoat; Smp. (C-$S_A$) 34 °C, Klp. ($S_A$-I) 41 °C.

Das als Ausgangsmaterial verwendete trans-4-(4′-[(R)-2-Octyl]oxycarbonyl-4-biphenylyl)cyclohexanol wurde wie folgt hergestellt:

(a) Ein Gemisch von 20 g 4-(4′-Amido-4-biphenylyl)cyclohexanon,200 ml 50%iger Schwefelsäure und 400 ml Essigsäure wurde über Nacht auf eine Temperatur von 115 °C erwärmt. Das gekühlte Reaktionsgemisch wurde auf Eiswasser gegossen und der weisse Niederschlag abfiltriert. Der Filterrückstand wurde mit Wasser gewaschen und anschliessend aus Aethanol umkristallisiert. Dies ergab 9,9 g 4-(4′-Carboxy-4-biphenylyl)cyclohexanon.

(b) Ein Gemisch von 2 g 4-(4′-Carboxy-4-biphenylyl)cyclohexanon, 0,9 g R-(-)-2-Octanol, 2,0 g N,N′-Dicyclohexylcarbodiimid, 1 mg 4-(Dimethylamino)pyridin und 80 ml Dichlormethan wurde in analoger Weise zu Beispiel 1 umgesetzt. Der Rückstand wurde an Kieselgel mit Hexan/Aethylacetat (Vol. 7:3) chromatographisch gereinigt. Dies ergab 1,5 g 4-(4′-[(R)-2-Octyl]oxycarbonyl-4-biphenylyl)cyclohexanon.

(c) Eine Suspension von 0,14 g Natriumborhydrid in 10 ml 1,2-Dimethoxyäthan wurde bei 0 °C mit einer Lösung von 4-(4′-[(R)-2-Octyl]oxycarbonyl-4-biphenylyl)cyclohexanon in 10 ml 1,2-Dimethoxyäthan versetzt. Das Reaktionsgemisch wurde noch 30 Minuten bei Raumtemperatur gerührt und dann mit 25%iger Salzsäure versetzt. Das Reaktionsgemisch wurde dreimal mit je 50ml Diäthyläther extrahiert. Die vereinigten organischen Phasen wurden mit Natriumhydrogencarbonat-Lösung und mehrmals mit Wasser gewaschen und anschliessend über Magnesiumsulfat getrocknet, filtriert und eingeengt. Der Rückstand wurde zweimal aus Hexan umkristallisiert und ergab 0,8 g trans-4-(4′-[(R)-2-Octyl]oxycarbonyl-4-biphenylyl)cyclohexanol.

In analoger Weise können die folgenden Verbindungen hergestellt werden:

trans-4-(4′-[(R)-2-Octyl]oxycarbonyl-4-biphenylyl)cyclohexyl-(S)-2-fluorpropanoat;
trans-4-(4′-[(R)-2-Octyl]oxycarbonyl-4-biphenylyl)cyclohexyl-(S)-2-fluorbutanoat;
trans-4-4′-[(R)-2-Octyl]oxycarbonyl-4-biphenylyl)cyclohexyl-(S)-2-fluorpentanoat;
trans-4-(4′-[(R)-2-Octyl]oxycarbonyl-4-biphenylyl)cyclohexyl-(S)-2-fluorheptanoat;
trans-4-(4′-[(R)-2-Octyl]oxycarbonyl-4-biphenylyl)cyclohexyl-(S)-2-fluoroctanoat;
trans-4-(4′-[(R)-2-Octyl]oxycarbonyl-4-biphenylyl)cyclohexyl-(S)-2-fluornonanoat;
trans-4-(4′-[(R)-2-Octyl]oxycarbonyl-4-biphenylyl)cyclohexyl-(S)-2-fluordecanoat;
trans-4-[2-(4′-[(R)-2-Octyl]oxycarbonyl-4-biphenylyl)äthyl]cyclohexyl-(S)-2-fluorpropanoat;
trans-4-[2-(4′-[(R)-2-Octyl]oxycarbonyl-4-biphenylyl)äthyl]cyclohexyl-(S)-2-fluorbutanoat;
trans-4-[2-(4′-[(R)-2-Octyl]oxycarbonyl-4-biphenylyl)äthyl]cyclohexyl-(S)-2-fluorpentanoat;
trans-4-[2-(4′-[(R)-2-Octyl]oxycarbonyl-4-biphenylyl)äthyl]cyclohexyl-(S)-2-fluorhexanoat;
trans-4-[2-(4′-[(R)-2-Octyl]oxycarbonyl-4-biphenylyl)äthyl]cyclohexyl-(S)-2-fluorheptanoat;
trans-4-[2-(4′-[(R)-2-Octyl]oxycarbonyl-4-biphenylyl)äthyl]cyclohexyl-(S)-2-fluoroctanoat;
trans-4-[2-(4′-[(R)-2-Octyl]oxycarbonyl-4-biphenylyl)äthyl]cyclohexyl-(S)-2-fluornonanoat;
trans-4-[2-(4′-[(R)-2-Octyl]oxycarbonyl-4-biphenylyl)äthyl]cyclohexyl-(S)-2-fluordecanoat.

## Beispiel 6

Die Verwendung der Verbindungen der Formel I in getilteten smektischen Phasen wird durch die in Tabelle 1 angegebenen Mischungen und durch Mischung M-3 veranschaulicht. Die Mengenangaben bedeuten Gew.-% der jeweiligen Komponente im Gesamtgemisch. Die mit D bezeichneten optisch aktiven Dotierstoffe der Formel I und die mit C bezeichneten weiteren Komponenten in Tabelle 1 bedeuten folgende Verbindungen:

C-1 = 4-Dodecyloxy-2-fluorbenzoesäure-4-[2-(trans-4-pentylcyclohexyl)äthyl]phenylester,
C-2 = 4-Dodecyloxy-2-fluorbenzoesäure-4-[(trans-4-pentylcyclohexyl)methoxy]phenylester,
C-3 = 4-Dodecyloxybenzoesäure-4-[2-(trans-4-pentylcyclohexyl)äthyl]phenylester,
C-4 = 4-Decyloxybenzoesäure-4-[2-(trans-4-pentylcyclohexyl)äthyl]phenylester,
C-5 = 4-Undecyloxybenzoesäure-4-[2-(trans-4-pentylcyclohexyl)äthyl]phenylester,
C-6 = 2-(4-Hexyloxyphenyl)-5-nonylpyrimidin,
C-7 = 2-(4-Nonyloxyphenyl)-5-nonylpyrimidin,
C-8 = 2-(4-Nonyloxyphenyl)-5-heptylpyrimidin,
C-9 = 2-(4-Octyloxyphenyl)-5-heptylpyrimidin,
C-10 = 2-(4-Heptyloxyphenyl)-5-heptylpyrimidin,
C-11 = 2-(4-Hexyloxyphenyl)-5-heptylpyrimidin,
C-12 = 2-(4-Decyloxyphenyl)-5-decylpyrimidin,
C-13 = 2-(4-Hexyloxyphenyl)-5-decylpyrimidin,

D-1 = trans-4-(4′-Decyl-4-biphenylyl)cyclohexyl-(S)-2-fluorhexanoat,
D-2 = trans-4-(4-[(trans-4-[(S)-2-fluorhexanoyloxy]cyclohexyl)methoxy]phenyl)cyclohexyl-(S)-2-fluorhexanoat.

Tabelle 1

| | M-1 | M-2 |
|---|---|---|
| C-1 | 16,32% | 14,44% |
| C-2 | 4,57% | 5,42% |
| C-3 | 5,86% | 5,23% |
| C-4 | 5,25% | 5,23% |
| C-5 | 3,30% | 4,87% |
| C-6 | 12,11% | 12,09% |
| C-7 | 21,79% | 20,58% |
| C-8 | 2,78% | 2,53% |
| C-9 | 7,59% | 6,23% |
| C-10 | 4,92% | 5,60% |
| C-11 | - | 4,51% |
| C-12 | 3,58% | - |
| C-13 | 3,63% | 3,52% |
| D-1 | 8,30% | - |
| D-2 | - | 9,75% |

Die Schmelzpunkte der Mischungen sind nur schwer zu bestimmen. Kristallisation oder hochgeordnete smektische Phasen konnten jedoch beim Abkühlen bis knapp unterhalb 0°C nicht beobachtet werden. Die Mischungen gemäss Tabelle 1 besitzen folgende Eigenschaften:

Mischung M-1

Smp. <0°C, Umwandlungen $S_C^*$ -$S_A$ 46,8°C, $S_A$-Ch 72,1°C,
Klp. (Ch-I) 89,2%.

Mischung M-2

Smp. <0°C, Umwandlungen $S_C^*$ -$S_A$ 18,3°C, $S_A$-Ch 70,7°C,
Klp. (Ch-I) 86,1°C.

Mischung M-3

3,53 Gew.-% trans-5-Hexyl-2-(4′-octyloxy-4-biphenylyl)-1,3-dioxan,
3,53 Gew.-% trans-5-Heptyl-2-(4′-heptyloxy-4-biphenylyl)-1,3-dioxan,
8,25 Gew.-% trans-5-Heptyl-2-(4′-octyloxy-4-biphenylyl)-1,3-dioxan,
8,20 Gew.-% trans-5-Nonyl-2-(4′-octyloxy-4-biphenylyl)-1,3-dioxan,
5,99 Gew.-% 2-(4-Hexyloxyphenyl)-5-octylpyrimidin,
12,73 Gew.-% 2-(4-Hexyloxyphenyl)-5-nonylpyrimidin,
7,72 Gew.-% 2-(4-Octyloxyphenyl)-5-heptylpyrimidin,
4,22 Gew.-% 2-(4-Nonyloxyphenyl)-5-heptylpyrimidin,
5,89 Gew.-% 2-(4-Nonyloxyphenyl)-5-octylpyrimidin,
25,44 Gew.-% 2-(4-Nonyloxyphenyl)-5-nonylpyrimidin,
5,00 Gew.-% trans-4-[4′-Decyl-4-biphenylyl)cyclohexyl-(S)-2-fluorhexanoat,
9,50 Gew.-% trans-4-[4-(2,3-Difluor-4-dodecyloxybenzoyloxy)phenyl]cyclohexyl-(S)-2-fluorhexanoat;
Smp. <0°C, Umwandlungen $S_C^*$-$S_A$ 61,1°C, $S_A$-Ch 83,7°C,
Klp. (Ch-I) 94,7°C; Ganghöhe 9 µm. In einer Zelle gemäss Appl. Phys. Lett. 36, 899 (1980) mit Plattenabstand 2 µm und einer Rechteckspannung von 20 V (peak to peak) ergab das Gemisch bei 25°C eine Ansprechzeit von 90 µs.

**Patentansprüche**

**1.** Optisch aktive Verbindungen der allgemeinen Formel

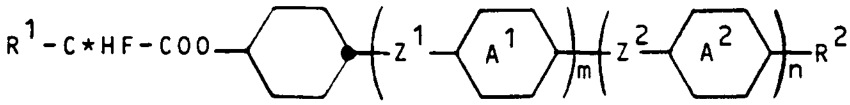

I

worin m für die Zahl 1 steht und $R^2$ eine unsubstituierte oder mit Halogen substituierte Alkyl- oder Alkenylgruppe bezeichnet, in welcher gegebenenfalls eine Methylengruppe durch Sauerstoff ersetzt ist und/oder gegebenenfalls eine Methylengruppe durch eine Estergruppe -COO- oder -OOC- ersetzt ist; oder m für die Zahl 0 oder 1 steht und $R^2$ eine Gruppe der allgemeinen Formel

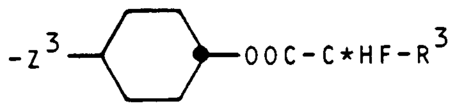

II

bezeichnet; $R^1$ und $R^3$ unabhängig voneinander eine Alkyl- oder Alkenylgruppe darstellen, in welcher gegebenenfalls eine Methylengruppe durch Sauerstoff ersetzt ist; $Z^1$, $Z^2$ und $Z^3$ unabhängig voneinander eine einfache Kovalenzbindung, $-CH_2O-$, $-OCH_2-$, $-CH_2CH_2-$, -COO- oder -OOC- bedeuten; n für die Zahl 0 oder 1 steht; die Ringe $A^1$ und $A^2$ unabhängig voneinander unsubstituiertes oder mit Halogen, Cyano und/oder Methyl substituiertes 1,4-Phenylen bedeuten, in welchem gegebenenfalls 1 oder 2 CH-Gruppen durch Stickstoff ersetzt sind; und $C^*$ ein chirales Kohlenstoffatom bezeichnet; mit der Massgabe, dass nicht zugleich $R^1$ eine Alkylgruppe, m und n die Zahl 1, $Z^1$ die Gruppe $-CH_2CH_2-$ oder $-CH_2O-$ und Ring $A^1$, $Z^2$, Ring $A^2$ und $R^2$ zusammen 4-(5-Alkyl-2-pyrimidinyl)phenyl bedeuten.

**2.** Verbindungen nach Anspruch 1, dadurch gekennzeichnet, dass die Ringe $A^1$ und $A^2$ unabhängig voneinander 1,4-Phenylen, Fluor-1,4-phenylen, Chlor-1,4-phenylen, Brom-1,4-phenylen, Cyano-1,4-phenylen, 2,3-Difluor-1,4-phenylen, Pyridin-2,5-diyl oder Pyrimidin-2,5-diyl bedeuten.

**3.** Verbindungen nach Anspruch 1 oder 2, gekennzeichnet durch die allgemeinen Formeln

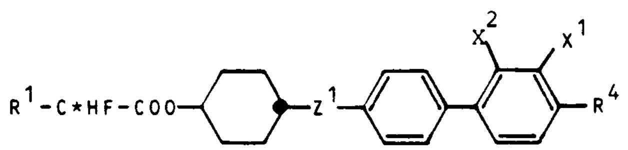

I-1

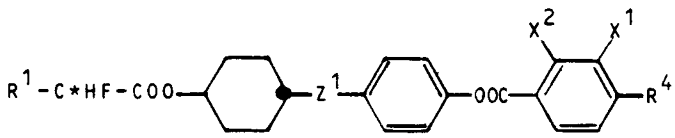

I-2

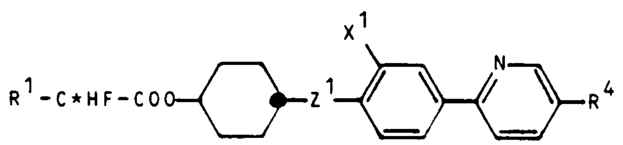

I-3

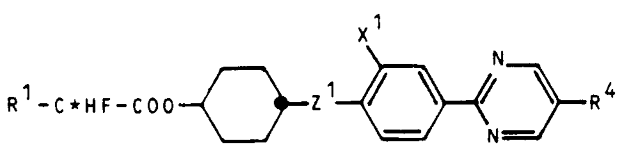

I-4

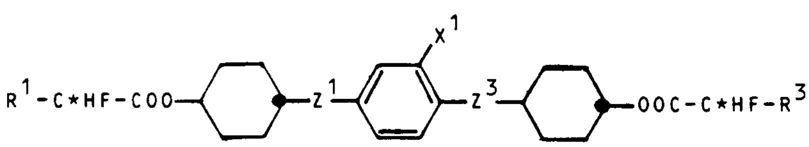

I-5

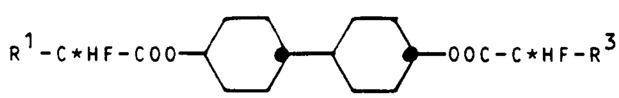

I-6

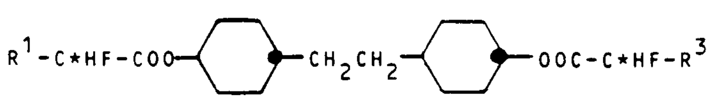

I-7

worin $R^1$, $R^3$, $Z^1$, $Z^3$ und $C^*$ die in Anspruch 1 gegebenen Bedeutungen haben; $R^4$ eine unsubstituierte oder mit Halogen substituierte Alkyl- oder Alkenylgruppe bezeichnet, in welcher gegebenenfalls eine Methylengruppe durch Sauerstoff ersetzt ist und/oder gegebenenfalls eine Methylengruppe durch eine Estergruppe -COO- oder -OOC-ersetzt ist, $X^1$ und $X^2$ Wasserstoff, Fluor, Chlor, Brom, Cyano oder Methyl bedeuten; mit der Massgabe, dass in Formel I-4 $X^1$ Fluor, Chlor, Brom, Cyano oder Methyl bezeichnet, wenn $Z^1$ $-CH_2CH_2$ - oder $-CH_2O-$ darstellt.

**4.** Verbindungen nach einem der Ansprüche 1 bis 3, dadurch gekennzeichnet, dass $Z^1$ eine einfache Kovalenzbindung, $-CH_2O-$, $-CH_2CH_2-$ oder -COO- und $Z^3$ eine einfache Kovalenzbindung, $-OCH_2-$, $-CH_2CH_2-$ oder -OOC- bedeuten.

**5.** Verbindungen nach einem der Ansprüche 1 bis 4, dadurch gekennzeichnet, dass $R^1$ Alkyl, Alkenyl, Alkoxyalkyl oder Alkenyloxyalkyl mit bis zu 15 Kohlenstoffatomen , vorzugsweise $C_3$-$C_{15}$-Alkyl bedeutet.

**6.** Verbindungen nach einem der Ansprüche 1 bis 5, dadurch gekennzeichnet, dass $R^2$ eine Gruppe der Formel II gezeichnet, und $R^3$ Alkyl, Alkenyl, Alkoxyalkyl oder Alkenyloxyalkyl mit bis zu 15 Kohlenstoffatomen bedeutet.

**7.** Verbindungen nach einem der Ansprüche 1 bis 5, dadurch gekennzeichnet, dass $R^2$ einen Rest $R^4$ bezeichnet und $R^4$ Alkyl, Alkenyl, Alkoxy, Alkenyloxy, Haloalkoxy, Alkoxycarbonyl oder Haloalkanoyloxy mit bis zu 15 Kohlenstoffatomen bedeutet.

**8.** Verfahren zur Herstellung von Verbindungen der in Anspruch 1 definierten Formel I, dadurch gekennzeichnet, dass man eine optische aktive $\alpha$-Fluorcarbonsäure der allgemeinen Formel

$R^1$-$C^*$HF-COOH III

und ein Cyclohexanol-Derivat der allgemeinen Formel

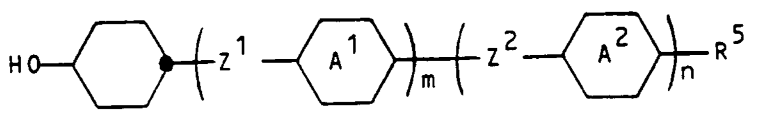

IV

worin m für die Zahl 1 steht und $R^5$ eine unsubstituierte oder mit Halogen substituierte Alkyl- oder Alkenylgruppe bezeichnet, in welcher gegebenenfalls eine Methylengruppe durch Sauerstoff ersetzt ist und/oder gegebenenfalls eine Methylengruppe durch eine Estergruppe -COO- oder -OOC- ersetzt ist; oder m für die Zahl 0 oder 1 steht und $R^5$ eine Gruppe der allgemeinen Formel

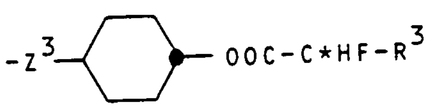

II

oder

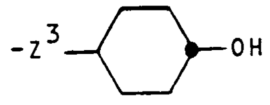

V

bezeichnet; $R^1$ und $R^3$ unabhängig voneinander eine Alkyl- oder Alkenylgruppe darstellen, in welcher gegebenenfalls eine Methylengruppe durch Sauerstoff ersetzt ist; $Z^1$, $Z^2$ und $Z^3$ unabhängig voneinander eine einfache Kovalenzbindung, $-CH_2O-$, $-OCH_2-$, $-CH_2CH_2-$, -COO- oder -OOC- bedeuten; n für die Zahl 0 oder 1 steht; die Ringe $A^1$ und $A^2$ unabhängig voneinander unsubstituiertes oder mit Halogen, Cyano und/oder Methyl substituiertes 1,4-Phenylen bedeuten, in welchem gegebenenfalls 1 oder 2 CH-Gruppen durch Stickstoff ersetzt sind; und $C^*$ ein chirales Kohlenstoffatom bezeichnet; mit der Massgabe, dass nicht zugleich $R^1$ eine Alkylgruppe, m und n die Zahl 1, $Z^1$ die Gruppe $-CH_2CH_2-$ oder $-CH_2O-$ und Ring $A^1$, $Z^2$, Ring $A^2$ und $R^5$ zusammen 4-(5-Alkyl-2-pyrimidinyl)phenyl bedeuten,
oder geeignete Derivate dieser Verbindungen verestert.

**9.** Flüssigkristallines Gemisch mit mindestens 2 Komponenten, dadurch gekennzeichnet, dass mindestens eine Komponente eine optische aktive Verbindung gemäss einem der Ansprüche 1 bis 7 ist.

**10.** Verwendung der Verbindungen der in Anspruch 1 definierten Formel I für elektro-optische Zwecke.

## Claims

**1.** Optically active compounds of the general formula

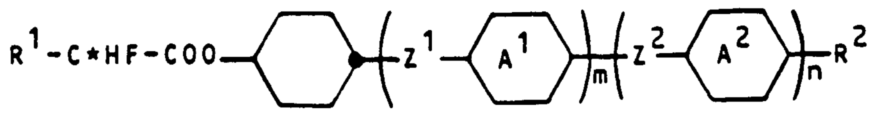

I

wherein m stands for the number 1 and $R^2$ signifies an unsubstituted or halogen-substituted alkyl or alkenyl group in which optionally one methylene group is replaced by oxygen and/or optionally one methylene group is replaced by an ester group -COO- or -OOC-; or m stands for the number 0 or 1 and $R^2$ denotes a group of the general formula

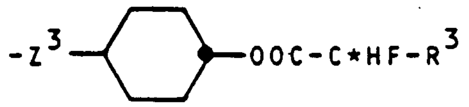

II;

$R^1$ and $R^3$ each independently represent an alkyl or alkenyl group in which optionally one methylene group is replaced by oxygen; $Z^1$, $Z^2$ and $Z^3$ each independently signify a single covalent bond. $-CH_2O-$, $-OCH_2-$, $-CH_2CH_2-$, -COO- or -OOC-; n stands for the number 0 or 1; rings $A^1$ and $A^2$ each independently signify unsubstituted or halogen-, cyano- and/or methyl-substituted 1,4-phenylene in which optionally 1 CH group or 2 CH groups is/are replaced by nitrogen; and C* denotes a chiral carbon atom; with the proviso that simultaneously $R^1$ does not signify an alkyl group, m and n do not signify the number 1, $Z^1$ does not signify the group $-CH_2CH_2-$ or or $-CH_2O-$ and ring $A^1$, $Z^2$, ring $A^2$ and $R^2$ together do not signify 4-(5-alkyl-2-pyrimidinyl)-phenyl.

**2.** Compounds according to claim 1, characterized in that rings $A^1$ and $A^2$ each independently signify 1,4-phenylene, fluoro-1,4-phenylene, chloro-1,4-phenylene, bromo-1,4-phenylene, cyano-1,4-phenylene, 2,3-difluoro-1,4-phenylene, pyridine-2,5-diyl or pyrimidine-2,5-diyl.

**3.** Compounds according to claim 1 or 2, characterized by the general formulae

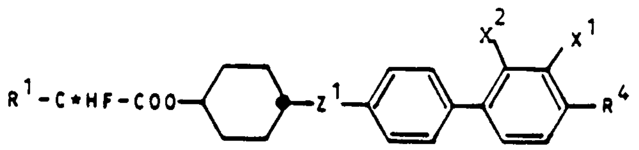

I-1

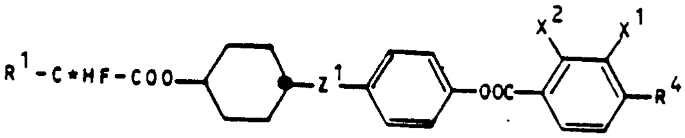

I-2

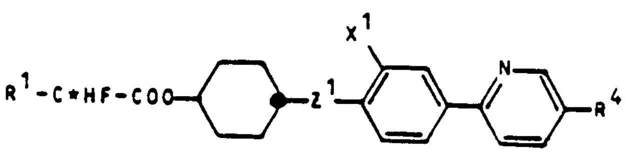

I-3

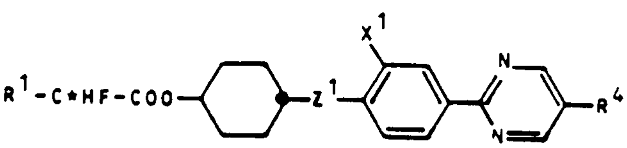

I-4

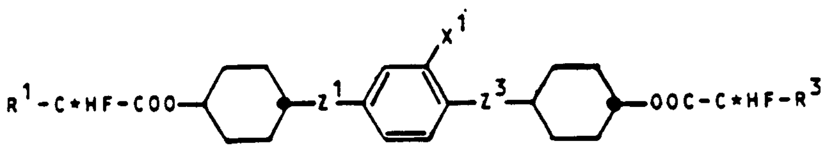

I-5

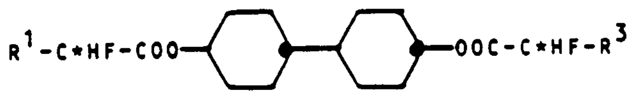

I-6

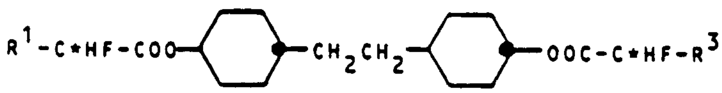

I-7

wherein $R^1$, $R^3$, $Z^1$, $Z^3$ and C* have the significances given in claim 1; $R^4$ represents an unsubstituted or halogen-substituted alkyl or alkenyl group in which optionally one methylene group is replaced by oxygen and/or optionally one methylene group is replaced by the ester group -COO- or -OOC-, $X^1$ and $X^2$ signify hydrogen, fluorine, chlorine, bromine, cyano or methyl; with the proviso that in formula I-4 $X^1$ denotes fluorine, chlorine, bromine, cyano or methyl when $Z^1$ represents $-CH_2CH_2-$ or $-CH_2O-$.

**4.** Compounds according to any one of claims 1 to 3, characterized in that $Z^1$ signifies a single covalent bond, $-CH_2O-$, $-CH_2CH_2-$ or -COO- and $Z^3$ signifies a single covalent bond, $-OCH_2-$, $-CH_2CH_2-$ or -OOC-.

**5.** Compounds according to any one of claims 1 to 4, characterized in that $R^1$ signifies alkyl, alkenyl, alkoxyalkyl or alkenyloxyalkyl with up to 15 carbon atoms, preferably $C_3$-$C_{15}$-alkyl.

**6.** Compounds according to any one of claims 1 to 5, characterized in that $R^2$ denotes a group of formula II and $R^3$ signifies alkyl, alkenyl, alkoxyalkyl or alkenyloxyalkyl with up to 15 carbon atoms.

**7.** Compounds according to any one of claims 1 to 5, characterized in that $R^2$ denotes a residue $R^4$ and $R^4$ signifies alkyl, alkenyl, alkoxy, alkenyloxy, haloalkoxy, alkoxycarbonyl or haloalkanoyloxy with up to 15 carbon atoms.

**8.** A process for the manufacture of compounds of formula I defined in claim 1, characterized by esterifying an optically active α-fluorocarboxylic acid of the general formula

$R^1$-C*HF-COOH III

and a cyclohexanol derivative of the general formula

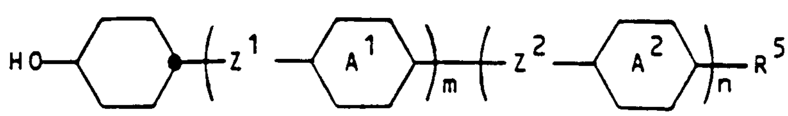

IV

wherein m stands for the number 1 and $R^5$ denotes an unsubstituted or halogen-substituted alkyl or alkenyl group in which optionally one methylene group is replaced by oxygen and/or optionally one methylene group is replaced by an ester group -COO- or -OOC-; or m stands for the number 0 or 1 and $R^5$ denotes a group of the general formula

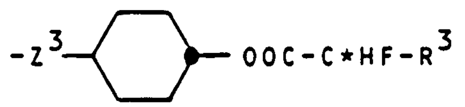

II

or

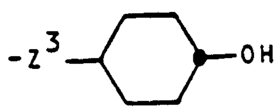

V;

$R^1$ and $R^3$ each independently represent an alkyl or alkenyl group in which optionally one methylene group is replaced by oxygen; $Z^1$, $Z^2$ and $Z^3$ each independently signify a single covalent bond, $-CH_2O-$, $-OCH_2-$, $-CH_2CH_2-$, -COO- or -OOC-; n stands for the number 0 or 1; rings $A^1$ and $A^2$ each independently signify unsubstituted or halogen-, cyano- and/or methyl-substituted 1,4-phenylene in which optionally 1 CH group or 2 CH groups is/are replaced by nitrogen; and C* denotes a chiral carbon atom; with the proviso that simultaneously $R^1$ does not signify an alkyl group, m and n do not signify the number 1, $Z^1$ does not signify the group $-CH_2CH_2-$ or $-CH_2O-$ and ring $A^1$, $Z^2$, ring $A^2$ and $R^5$ together do not signify 4-(5-alkyl-2-pyrimidinyl)-phenyl,
or suitable derivatives of these compounds.

**9.** A liquid crystalline mixture having at least 2 components, characterized in that at least one component is an optically active compound in accordance with any one of claims 1 to 7.

**10.** The use of the compounds of formula I defined in claim 1 for electro-optical purposes.

## Revendications

**1.** Composés, possédant l'activité optique, de formule générale

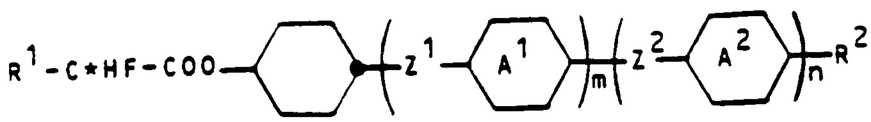

I

dans laquelle m est égal à 1 et $R^2$ représente un groupe alkyle ou alcényle non substitué ou substitué par des halogènes et dans lesquels le cas échéant un groupe méthylène est remplacé par l'oxygène et/ou le cas échéant un groupe méthylène est remplacé par un groupe ester -COO- ou -OOC- ; ou bien m est égal à 0 ou 1 et $R^2$ représente un groupe de formule générale

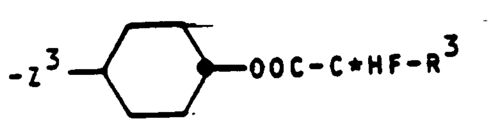

II

$R^1$ et $R^3$ représentent chacun, indépendamment l'un de l'autre, un groupe alkyle ou alcényle dans lequel le cas échéant un groupe méthylène est remplacé par l'oxygène ; $Z^1$, $Z^2$ et $Z^3$ représentent chacun, indépendamment les uns des autres, une liaison covalente simple, $-CH_2O-$, $-OCH_2-$, $-CH_2CH_2-$,

-COO- ou -OOC-; n est égal à 0 ou 1; les cycles $A^1$ et $A^2$, indépendamment l'un de l'autre, sont des cycles 1,4-phénylène non substitués ou substitués par des halogènes, des groupes cyano et/ou méthyle, et dans lesquels le cas échéant 1 ou 2 groupes CH sont remplacés par l'azote ; et C* représente un atome de carbone chiral; à l'exclusion des composés pour lesquels, simultanément, $R^1$ représente un groupe alkyle, m et n sont égaux à 1, $Z^1$ représente un groupe $-CH_2CH_2-$ ou $-CH_2O-$ et et le cycle $A^1$, $Z^2$, le cycle $A^2$ et $R^2$ forment ensemble un groupe 4-(5-alkyl-2-pyrimidinyl)-phényle.

2. Composés selon revendication 1, caractérisés en ce que les cycles $A^1$ et $A^2$ sont chacun, indépendamment l'un de l'autre, un cycle 1,4-phénylène, fluoro-1,4-phénylène, chloro-1,4-phénylène, bromo-1,4-phénylène, cyano-1,4-phénylène, 2,3-difluoro-1,4-phénylène, pyridine-2,5-diyle ou pyrimidine-2,5-diyle.

3. Composés selon revendication 1 ou 2, caractérisés par les formules générales

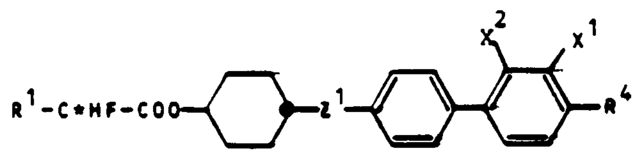

I-1

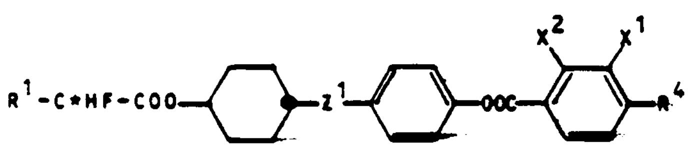

I-2

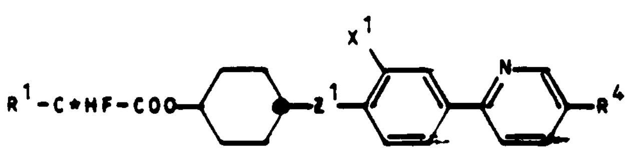

, I-3

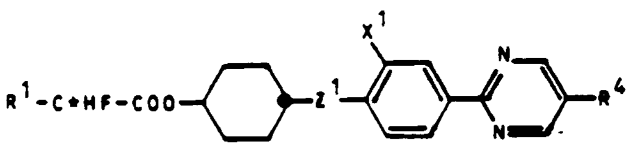

I-4

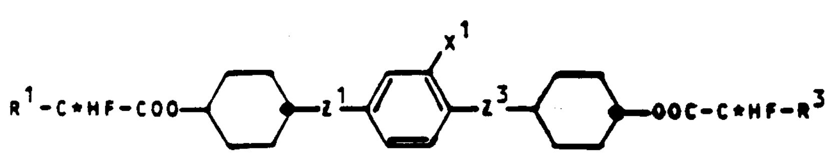

I-5

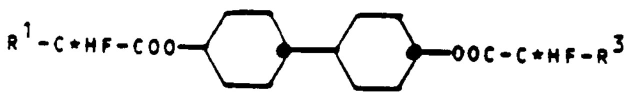

I-6

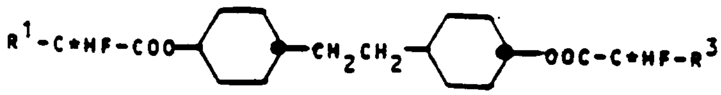

I-7

dans lesquelles $R^1$, $R^3$, $Z^1$, $Z^3$ et C* ont les significations indiquées dans la revendication 1 ; $R^4$ représente un groupe alkyle ou alcényle non substitué ou substitué par des halogènes et dans lequel le cas échéant un groupe méthylène est remplacé par l'oxygène et/ou le cas échéant un groupe méthylène est remplacé par un groupe ester -COO- ou -OOC-, $X^1$ et $X^2$ représentent l'hydrogène, le fluor, le chlore, le brome, des groupes cyano ou méthyle ; sous réserve que, dans la formule 1-4, $X^1$ représente le fluor, le chlore, le brome, un groupe cyano ou méthyle lorsque $Z^1$ représente $-CH_2CH_2-$ ou $-CH_2O-$.

**4.** Composés selon une des revendications 1 à 3, caractérisés en ce que $Z^1$ représente une liaison covalente simple, un groupe $-CH_2O-$, $-CH_2CH_2-$ ou ou -COO- et $Z^3$ représente une liaison covalente simple, $-OCH_2-$, $-CH_2CH_2-$ ou -OOC-.

**5.** Composés selon une des revendications 1 à 4, caractérisés en ce que $R^1$ représente un groupe alkyle, alcényle, alcoxyalkyle ou alcényloxyalkyle contenant jusqu'à 15 atomes de carbone, de préférence un groupe alkyle en C 3-C 15.

**6.** Composés selon une des revendications 1 à 5, caractérisés en ce que $R^2$ représente un groupe de formule II et $R^3$ représente un groupe alkyle, alcényle, alcoxyalkyle ou alcényloxyalkyle contenant jusqu'à 15 atomes de carbone.

**7.** Composés selon une des revendications 1 à 5, caractérisés en ce que $R^2$ représente un groupe $R^4$ et $R^4$ représente un groupe alkyle, alcényle, alcoxy, alcényloxy, halogénoalcoxy, alcoxycarbonyle ou halogénoalcanoyloxy contenant jusqu'à 15 atomes de carbone.

**8.** Procédé de préparation des composés de formule I définie dans la revendication 1, caractérisé en ce que l'on estérifie un acide alpha-fluorocarboxylique, possédant l'activité optique, de formule générale

$R^1$-C*HF-COOH III

par un dérivé du cyclohexanol de formule générale

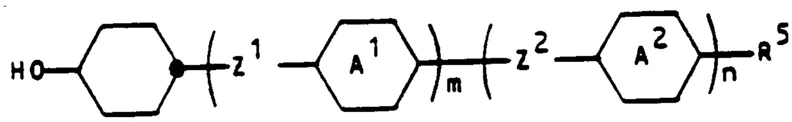

IV

dans lesquelles m est égal à 1 et $R^5$ représente un groupe alkyle ou alcényle non substitué ou substitué par des halogènes et dans lequel le cas échéant un groupe méthylène est remplacé par l'oxygène et/ou le cas échéant un groupe méthylène est remplacé par un groupe ester -COO- ou -OOC- ; ou bien m est égal à 0 ou 1 et $R^5$ représente un groupe de formule générale

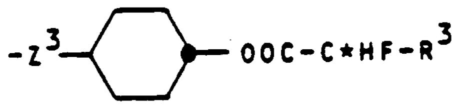

II

ou

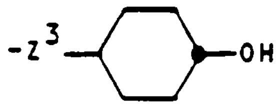

V

$R^1$ et $R^3$ représentent chacun, indépendamment l'un de l'autre, un groupe alkyle ou alcényle dans lequel le cas échéant un groupe méthylène est remplacé par l'oxygène ; $Z^1$, $Z^2$ et $Z^3$ représentent chacun, indépendamment les uns des autres, une liaison covalente simple, un groupe $-CH_2O-$, $-OCH_2-$, $-CH_2CH_2-$, -COO- ou ou -OOC- ; n est égal à 0 ou 1 ; les cycles $A^1$ et $A^2$ sont chacun, indépendamment l'un de l'autre, des cycles 1,4-phénylène non substitués ou substitués par des halogènes, des groupes cyano et/ou méthyle, et dans lesquels le cas échéant 1 ou 2 groupes CH sont remplacés par l'azote ; et C* représente un atome de carbone chiral ; à l'exclusion des composés pour lesquels, simultanément, $R^1$ représente un groupe alkyle, m et n sont égaux à 1, $Z^1$ représente un groupe $-CH_2CH_2-$ ou $-CH_2O-$ et le cycle $A^1$, $Z^2$, le cycle $A^2$ et $R^5$ forment ensemble un groupe 4-(5-alkyl-2-pyrimidinyl)-phényle,

ou par un dérivé approprié d'un tel composé.

**9.** Mélange à cristaux liquides à au moins deux composants, caractérisé en ce que l'un au moins des composants consiste en un composé, possédant l'activité optique, selon une des revendications 1 à 7.

**10.** Utilisation des composés de formule I définie dans la revendication 1 dans des applications électro-optiques.